(19) Europäisches Patentamt
European Patent Office
Office européen des brevets

(11) **EP 4 353 258 A1**

(12) **EUROPEAN PATENT APPLICATION**
published in accordance with Art. 153(4) EPC


(43) Date of publication:
**17.04.2024 Bulletin 2024/16**

(21) Application number: **22804761.9**

(22) Date of filing: **19.05.2022**

(51) International Patent Classification (IPC):
***A61K 45/00*** $^{(2006.01)}$ ***A61K 39/395*** $^{(2006.01)}$
***A61P 35/00*** $^{(2006.01)}$ ***A61P 43/00*** $^{(2006.01)}$
***C07K 16/28*** $^{(2006.01)}$ ***C07K 16/46*** $^{(2006.01)}$
***C12N 15/13*** $^{(2006.01)}$ ***C12P 21/08*** $^{(2006.01)}$

(52) Cooperative Patent Classification (CPC):
**A61K 39/395; A61K 45/00; A61P 35/00;**
**A61P 43/00; C07K 16/28;** C07K 16/00; C07K 16/46

(86) International application number:
**PCT/JP2022/020881**

(87) International publication number:
**WO 2022/244852 (24.11.2022 Gazette 2022/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.05.2021 JP 2021084836**



(71) Applicant: **National University Corporation University Of Toyama**
**Toyama-shi, Toyama 930-8555 (JP)**

(72) Inventors:
- **TOBE, Kazuyuki**
  **Toyama-shi, Toyama 930-0194 (JP)**
- **KADO, Tomonobu**
  **Toyama-shi, Toyama 930-0194 (JP)**
- **OZAWA, Tatsuhiko**
  **Toyama-shi, Toyama 930-0194 (JP)**
- **MORI, Hisashi**
  **Toyama-shi, Toyama 930-0194 (JP)**
- **YOSHIDA, Tomoyuki**
  **Toyama-shi, Toyama 930-0194 (JP)**
- **IGARASHI, Yoshiko**
  **Toyama-shi, Toyama 930-0194 (JP)**

(74) Representative: **Hasegawa, Kan**
**Patentanwaltskanzlei Hasegawa**
**Untere Hauptstraße 56**
**85354 Freising (DE)**


Remarks:
The complete document including Reference Table(s) and the Sequence Listing(s) can be downloaded from the EPO website

(54) **PHARMACEUTICAL COMPOSITION FOR PREVENTING OR TREATING CANCER**


(57) An object of the present invention is to reveal association of macrophages with the development/progression of a cancer, and to provide a novel pharmaceutical composition for the prevention or treatment of a cancer. The present invention provides a pharmaceutical composition for the prevention or treatment of a cancer; a composition for killing fibroblasts locating adjacent to a tumor tissue; a composition for promoting infiltration of cytotoxic T cells into a tumor tissue; a composition for suppressing the expression of a gene(s) in fibroblasts, which gene(s) suppress(es) infiltration of cytotoxic T cells into a tumor; a composition for promoting the growth of cytotoxic T cells; a composition for enhancing the cytotoxic activity of cytotoxic T cells; and a composition for enhancing the expression of a gene(s) associated with the cytotoxicity of cytotoxic T cells; containing, as an effective component, a substance having an ability to kill CD206-positive M2 macrophages.


Fig. 1

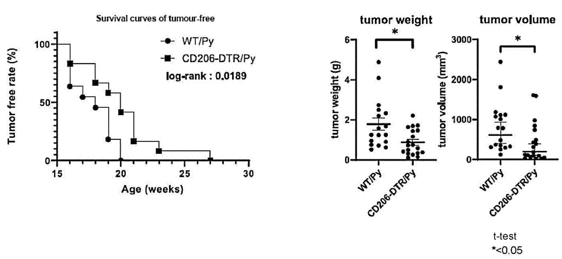


EP 4 353 258 A1

## Description

TECHNICAL FIELD

The present invention relates, for example, to a pharmaceutical composition for the prevention or treatment of a cancer, the pharmaceutical composition comprising, as an effective component, a substance having an ability to kill CD206-positive M2 macrophages.

BACKGROUND ART

As anticancer drugs to be used for the cancer treatment, there are a variety of anticancer drugs based on various mechanisms. However, since the development of definitive anticancer drugs has not yet been done, development of more powerful anticancer drugs and cancer treatment aids has been demanded. Further, since there are cases where resistance to anticancer drugs are formed to prevent effectiveness of the anticancer drugs, development of new anticancer drugs has been demanded.

Macrophages are a type of major cells in the cancer microenvironment. There are macrophages that contribute to the growth and metastasis of cancer cells (Non-Patent Document 1). Although reduction of macrophages protective for cancer cells has been thought to be beneficial for cancer treatment, such reduction has not yet been utilized as a common treatment.

In the analysis of macrophages, they have been roughly divided into M1 macrophages and M2 macrophages using cell surface markers. Although there is a report suggesting that M2 macrophages have a function protective for cancer cells, macrophages are cells showing diversity. Thus, it has now been thought that the simple dichotomous classification of macrophages is inappropriate, and that they need to be divided into smaller groups when they are analyzed.

CD206 is a marker of M2 macrophages. However, studies using in vivo models have not been sufficiently carried out regarding whether or not CD206-positive M2 macrophages are actually involved, and what roles they play, in the development/progression of cancers. Furthermore, the molecular mechanisms of the development/progression have been unclear.

PRIOR ART DOCUMENT

[Non-patent Document]

[Non-Patent Document 1] Nat Rev Immunol. 19(6): 369-382, 2019

SUMMARY OF THE INVENTION

TECHNICAL PROBLEM

In view of the above circumstances, an object of the present invention is to reveal association of macrophages with the development/progression of a cancer, and to provide a novel pharmaceutical composition for the prevention or treatment of a cancer.

SOLUTION TO PROBLEM

The present inventors intensively studied in order to reveal association of macrophages with the development/progression of a cancer, and to provide a novel pharmaceutical composition for the prevention or treatment of a cancer. More specifically, the present inventors studied the nature and roles of CD206-positive M2 macrophages in the development/progression of a cancer using a unique mouse model. As a result, CD206-positive M2 macrophages were found to contribute positively to the development/progression of a cancer in an in vivo model. It was also found that removal of CD206-positive M2 macrophages enables induction of anticancer responses. It was also discovered that removal of CD206-positive M2 macrophages enables promotion of various anti-tumor immune responses. Based on such discoveries, the present invention was completed.

More specifically, the present invention can be summarized as follows.

[1] A composition for killing fibroblasts locating adjacent to a tumor tissue, wherein the composition comprising, as an effective component, a substance having an ability to kill CD206-positive M2 macrophages.

[2] A composition for promoting infiltration of cytotoxic T cells into a tumor tissue, wherein the composition comprising, as an effective component, a substance having an ability to kill CD206-positive M2 macrophages.
[3] The composition for promoting infiltration of cytotoxic T cells into a tumor tissue according to [2], wherein the promotion of infiltration of the cytotoxic T cells into the tumor tissue involves reduction of fibroblasts locating adjacent to the tumor tissue.
[4] A composition for suppressing expression of one or more genes selected from Acta2, CXCL12, and CXCL14 on fibroblasts locating adjacent to a tumor tissue, wherein the composition comprising, as an effective component, a substance having an ability to kill CD206-positive M2 macrophages.
[5] A composition for promoting growth of cytotoxic T cells, wherein the composition comprising, as an effective component, a substance having an ability to kill CD206-positive M2 macrophages.
[6] A composition for enhancing cytotoxic activity of cytotoxic T cells, wherein the composition comprising, as an effective component, a substance having an ability to kill CD206-positive M2 macrophages.
[7] A composition for enhancing expression of one or more genes selected from TNF$\alpha$, IFN$\gamma$, and Gzmb, wherein the composition comprising, as an effective component, a substance having an ability to kill CD206-positive M2 macrophages. [0010]
[8] A pharmaceutical composition for prevention or treatment of a cancer, wherein the pharmaceutical composition comprising, as an effective component, a substance having an ability to kill CD206-positive M2 macrophages.
[9] The pharmaceutical composition for the prevention or treatment of a cancer according to [8], wherein the substance having an ability to kill CD206-positive M2 macrophages is an anti-CD206 antibody or a functional fragment thereof.
[10] The pharmaceutical composition for the prevention or treatment of a cancer according to [9], wherein the antibody is an antibody selected from the following:

(a) an antibody containing:

heavy-chain HCDR1 having the amino acid sequence of SEQ ID NO:27;
heavy-chain HCDR2 having the amino acid sequence of SEQ ID NO:28;
heavy-chain HCDR3 having the amino acid sequence of SEQ ID NO:29;
light-chain LCDR1 having the amino acid sequence of SEQ ID NO:30;
light-chain LCDR2 having the amino acid sequence of YTS; and
light-chain LCDR3 having the amino acid sequence of SEQ ID NO:31;

(b) an antibody containing:

heavy-chain HCDR1 having the amino acid sequence of SEQ ID NO:37;
heavy-chain HCDR2 having the amino acid sequence of SEQ ID NO:38;
heavy-chain HCDR3 having the amino acid sequence of SEQ ID NO:39;
light-chain LCDR1 having the amino acid sequence of SEQ ID NO:40;
light-chain LCDR2 having the amino acid sequence of LMS; and
light-chain LCDR3 having the amino acid sequence of SEQ ID NO:41;

(c) an antibody containing:

heavy-chain HCDR1 having the amino acid sequence of SEQ ID NO:42;
heavy-chain HCDR2 having the amino acid sequence of SEQ ID NO:43;
heavy-chain HCDR3 having the amino acid sequence of SEQ ID NO:44;
light-chain LCDR1 having the amino acid sequence of SEQ ID NO:45;
light-chain LCDR2 having the amino acid sequence of RAS; and
light-chain LCDR3 having the amino acid sequence of SEQ ID NO:46;

(d) an antibody containing:

heavy-chain HCDR1 having the amino acid sequence of SEQ ID NO:47;
heavy-chain HCDR2 having the amino acid sequence of SEQ ID NO:48;
heavy-chain HCDR3 having the amino acid sequence of SEQ ID NO:49;
light-chain LCDR1 having the amino acid sequence of SEQ ID NO:50;
light-chain LCDR2 having the amino acid sequence of FAS; and
light-chain LCDR3 having the amino acid sequence of SEQ ID NO:51;

(e) an antibody containing:

heavy-chain HCDR1 having the amino acid sequence of SEQ ID NO:52;
heavy-chain HCDR2 having the amino acid sequence of SEQ ID NO:53;
heavy-chain HCDR3 having the amino acid sequence of SEQ ID NO:54;
light-chain LCDR1 having the amino acid sequence of SEQ ID NO:55;
light-chain LCDR2 having the amino acid sequence of AAT; and
light-chain LCDR3 having the amino acid sequence of SEQ ID NO:56;

(f) an antibody containing:

heavy-chain HCDR1 having the amino acid sequence of SEQ ID NO:57;
heavy-chain HCDR2 having the amino acid sequence of SEQ ID NO:58;
heavy-chain HCDR3 having the amino acid sequence of SEQ ID NO:59;
light-chain LCDR1 having the amino acid sequence of SEQ ID NO:60;
light-chain LCDR2 having the amino acid sequence of YTS; and
light-chain LCDR3 having the amino acid sequence of SEQ ID NO:61;

(g) an antibody containing:

heavy-chain HCDR1 having the amino acid sequence of SEQ ID NO:62;
heavy-chain HCDR2 having the amino acid sequence of SEQ ID NO:63;
heavy-chain HCDR3 having the amino acid sequence of SEQ ID NO:64;
light-chain LCDR1 having the amino acid sequence of SEQ ID NO:65;
light-chain LCDR2 having the amino acid sequence of KAS; and
light-chain LCDR3 having the amino acid sequence of SEQ ID NO:66;

(h) an antibody containing:

heavy-chain HCDR1 having the amino acid sequence of SEQ ID NO:67;
heavy-chain HCDR2 having the amino acid sequence of SEQ ID NO:68;
heavy-chain HCDR3 having the amino acid sequence of SEQ ID NO:69;
light-chain LCDR1 having the amino acid sequence of SEQ ID NO:70;
light-chain LCDR2 having the amino acid sequence of YTS; and
light-chain LCDR3 having the amino acid sequence of SEQ ID NO:71;

(i) an antibody containing:

heavy-chain HCDR1 having the amino acid sequence of SEQ ID NO:77;
heavy-chain HCDR2 having the amino acid sequence of SEQ ID NO:78;
heavy-chain HCDR3 having the amino acid sequence of SEQ ID NO:79;
light-chain LCDR1 having the amino acid sequence of SEQ ID NO:80;
light-chain LCDR2 having the amino acid sequence of ATS; and
light-chain LCDR3 having the amino acid sequence of SEQ ID NO:81;

(j) an antibody containing:

heavy-chain HCDR1 having the amino acid sequence of SEQ ID NO:82;
heavy-chain HCDR2 having the amino acid sequence of SEQ ID NO:83;
heavy-chain HCDR3 having the amino acid sequence of SEQ ID NO:84;
light-chain LCDR1 having the amino acid sequence of SEQ ID NO:85;
light-chain LCDR2 having the amino acid sequence of RAN; and
light-chain LCDR3 having the amino acid sequence of SEQ ID NO:86; and

(k) an antibody containing:

heavy-chain HCDR1 having the amino acid sequence of SEQ ID NO:87;
heavy-chain HCDR2 having the amino acid sequence of SEQ ID NO:88;
heavy-chain HCDR3 having the amino acid sequence of SEQ ID NO:89;
light-chain LCDR1 having the amino acid sequence of SEQ ID NO:90;

light-chain LCDR2 having the amino acid sequence of YTS; and
light-chain LCDR3 having the amino acid sequence of SEQ ID NO:91.

[11] The pharmaceutical composition for the prevention or treatment of a cancer according to [9] or [10], wherein the antibody is an antibody selected from the following:

(A) an antibody containing:

a heavy-chain variable region having the amino acid sequence of SEQ ID NO:1, or a heavy-chain variable region having an amino acid sequence with a sequence identity of not less than 90% to the amino acid sequence and having a CD206-binding activity; and
a light-chain variable region having the amino acid sequence of SEQ ID NO:2, or a light-chain variable region having an amino acid sequence with a sequence identity of not less than 90% to the amino acid sequence and having a CD206-binding activity;

(B) an antibody containing:

a heavy-chain variable region having the amino acid sequence of SEQ ID NO:5, or a heavy-chain variable region having an amino acid sequence with a sequence identity of not less than 90% to the amino acid sequence and having a CD206-binding activity; and
a light-chain variable region having the amino acid sequence of SEQ ID NO:6, or a light-chain variable region having an amino acid sequence with a sequence identity of not less than 90% to the amino acid sequence and having a CD206-binding activity;

(C) an antibody containing:

a heavy-chain variable region having the amino acid sequence of SEQ ID NO:7, or a heavy-chain variable region having an amino acid sequence with a sequence identity of not less than 90% to the amino acid sequence and having a CD206-binding activity; and
a light-chain variable region having the amino acid sequence of SEQ ID NO:8, or a light-chain variable region having an amino acid sequence with a sequence identity of not less than 90% to the amino acid sequence and having a CD206-binding activity;

(D) an antibody containing:

a heavy-chain variable region having the amino acid sequence of SEQ ID NO:9, or a heavy-chain variable region having an amino acid sequence with a sequence identity of not less than 90% to the amino acid sequence and having a CD206-binding activity; and
a light-chain variable region having the amino acid sequence of SEQ ID NO:10, or a light-chain variable region having an amino acid sequence with a sequence identity of not less than 90% to the amino acid sequence and having a CD206-binding activity;

(E) an antibody containing:

a heavy-chain variable region having the amino acid sequence of SEQ ID NO: 11, or a heavy-chain variable region having an amino acid sequence with a sequence identity of not less than 90% to the amino acid sequence and having a CD206-binding activity; and
a light-chain variable region having the amino acid sequence of SEQ ID NO: 12, or a light-chain variable region having an amino acid sequence with a sequence identity of not less than 90% to the amino acid sequence and having a CD206-binding activity;

(F) an antibody containing:

a heavy-chain variable region having the amino acid sequence of SEQ ID NO: 13, or a heavy-chain variable region having an amino acid sequence with a sequence identity of not less than 90% to the amino acid sequence and having a CD206-binding activity; and
a light-chain variable region having the amino acid sequence of SEQ ID NO:14, or a light-chain variable region having an amino acid sequence with a sequence identity of not less than 90% to the amino acid sequence and having a CD206-binding activity;

(G) an antibody containing:

a heavy-chain variable region having the amino acid sequence of SEQ ID NO:15, or a heavy-chain variable region having an amino acid sequence with a sequence identity of not less than 90% to the amino acid sequence and having a CD206-binding activity; and
a light-chain variable region having the amino acid sequence of SEQ ID NO:16, or a light-chain variable region having an amino acid sequence with a sequence identity of not less than 90% to the amino acid sequence and having a CD206-binding activity;

(H) an antibody containing:

a heavy-chain variable region having the amino acid sequence of SEQ ID NO:17, or a heavy-chain variable region having an amino acid sequence with a sequence identity of not less than 90% to the amino acid sequence and having a CD206-binding activity; and
a light-chain variable region having the amino acid sequence of SEQ ID NO:18, or a light-chain variable region having an amino acid sequence with a sequence identity of not less than 90% to the amino acid sequence and having a CD206-binding activity;

(I) an antibody containing:

a heavy-chain variable region having the amino acid sequence of SEQ ID NO:21, or a heavy-chain variable region having an amino acid sequence with a sequence identity of not less than 90% to the amino acid sequence and having a CD206-binding activity; and
a light-chain variable region having the amino acid sequence of SEQ ID NO:22, or a light-chain variable region having an amino acid sequence with a sequence identity of not less than 90% to the amino acid sequence and having a CD206-binding activity;

(J) an antibody containing:

a heavy-chain variable region having the amino acid sequence of SEQ ID NO:25, or a heavy-chain variable region having an amino acid sequence with a sequence identity of not less than 90% to the amino acid sequence and having a CD206-binding activity; and
a light-chain variable region having the amino acid sequence of SEQ ID NO:26, or a light-chain variable region having an amino acid sequence with a sequence identity of not less than 90% to the amino acid sequence and having a CD206-binding activity; and

(K) an antibody containing:

a heavy-chain variable region having the amino acid sequence of SEQ ID NO:25, or a heavy-chain variable region having an amino acid sequence with a sequence identity of not less than 90% to the amino acid sequence and having a CD206-binding activity; and
a light-chain variable region having the amino acid sequence of SEQ ID NO:26, or a light-chain variable region having an amino acid sequence with a sequence identity of not less than 90% to the amino acid sequence and having a CD206-binding activity.

[12] A method of prevention or treatment of a cancer, wherein the method comprising the step of administering a substance having an ability to kill CD206-positive M2 macrophages to a subject in need of the prevention or treatment.
[13] Use of a substance having an ability to kill CD206-positive M2 macrophages, for the prevention or treatment of a cancer.
[14] Use of a substance having an ability to kill CD206-positive M2 macrophages, for the production of a pharmaceutical composition for the prevention or treatment of a cancer.
[15] A method of killing fibroblasts locating adjacent to a tumor tissue, wherein the method comprising the step of administering a substance having an ability to kill CD206-positive M2 macrophages to a subject.
[16] Use of a substance having an ability to kill CD206-positive M2 macrophages, for killing fibroblasts locating adjacent to a tumor tissue.
[17] Use of a substance having an ability to kill CD206-positive M2 macrophages, for the production of a composition for killing fibroblasts locating adjacent to a tumor tissue.

[18] A method of promoting infiltration of cytotoxic T cells into a tumor tissue, wherein the method comprising the step of administering a substance having an ability to kill CD206-positive M2 macrophages to a subject.

[19] Use of a substance having an ability to kill CD206-positive M2 macrophages, for promotion of infiltration of cytotoxic T cells into a tumor tissue.

[20] Use of a substance having an ability to kill CD206-positive M2 macrophages, for the production of a composition for promoting infiltration of cytotoxic T cells into a tumor tissue.

[21] A method of suppressing the expression of one or more genes selected from Acta2, CXCL12, and CXCL14 on fibroblasts locating adjacent to a tumor tissue, wherein the method comprising the step of administering a substance having an ability to kill CD206-positive M2 macrophages to a subject.

[22] Use of a substance having an ability to kill CD206-positive M2 macrophages, for suppressing the expression of one or more genes selected from Acta2, CXCL12, and CXCL14 on fibroblasts locating adjacent to a tumor tissue.

[23] Use of a substance having an ability to kill CD206-positive M2 macrophages, for the production of a composition for suppressing the expression of one or more genes selected from Acta2, CXCL12, and CXCL14 on fibroblasts locating adjacent to a tumor tissue.

[24] A method of promoting the growth of cytotoxic T cells, wherein the method comprising the step of administering a substance having an ability to kill CD206-positive M2 macrophages to a subject.

[25] Use of a substance having an ability to kill CD206-positive M2 macrophages, for promotion of the growth of cytotoxic T cells.

[26] Use of a substance having an ability to kill CD206-positive M2 macrophages, for the production of a composition for promoting the growth of cytotoxic T cells.

[27] A method of enhancing the cytotoxic activity of cytotoxic T cells, wherein the method comprising the step of administering a substance having an ability to kill CD206-positive M2 macrophages to a subject.

[28] Use of a substance having an ability to kill CD206-positive M2 macrophages, for enhancing the cytotoxic activity of cytotoxic T cells.

[29] Use of a substance having an ability to kill CD206-positive M2 macrophages, for the production of a composition for enhancing the cytotoxic activity of cytotoxic T cells.

[30] A method of enhancing the expression of one or more genes selected from TNF$\alpha$, IFN$\gamma$, and Gzmb, wherein the method comprising the step of administering a substance having an ability to kill CD206-positive M2 macrophages to a subject.

[31] Use of a substance having an ability to kill CD206-positive M2 macrophages, for enhancing the expression of one or more genes selected from TNF$\alpha$, IFN$\gamma$, and Gzmb.

[32] Use of a substance having an ability to kill CD206-positive M2 macrophages, for the production of a composition for enhancing the expression of one or more genes selected from TNF$\alpha$, IFN$\gamma$, and Gzmb.

## ADVANTAGEOUS EFFECTS OF THE INVENTION

The present invention revealed association of macrophages with the development/progression of a cancer, and provides a pharmaceutical composition for the prevention or treatment of a cancer, the pharmaceutical composition comprising, as an effective component, a substance having an ability to remove CD206-positive M2 macrophages. The present invention also provides a composition for killing fibroblasts locating adjacent to a tumor tissue, a composition for promoting infiltration of cytotoxic T cells into a tumor tissue, a composition for suppressing the expression of a gene(s) in fibroblasts, which gene(s) suppress(es) infiltration of cytotoxic T cells into a tumor, a composition for promoting the growth of cytotoxic T cells, a composition for enhancing the cytotoxic activity of cytotoxic T cells, and a composition for enhancing the expression of a gene(s) associated with the cytotoxicity of cytotoxic T cells, containing, as an effective component, a substance having an ability to remove CD206-positive M2 macrophages.

## BRIEF DESCRIPTION OF THE DRAWINGS

Fig. 1 is a diagram showing results of evaluation of the action of removal of CD206-positive M2 macrophages, on a tumor. The left panel shows the effect of the removal of CD206-positive M2 macrophages on the tumor incidence. The middle panel shows the effect of the removal of CD206-positive M2 macrophages on the tumor weight. The right panel shows the effect of the removal of CD206-positive M2 macrophages on the tumor volume.

Fig. 2 is a diagram showing results of evaluation of the action of removal of CD206-positive M2 macrophages on the number of CD8-positive T cells. The two left panels show results (drawing-substituting photographs) of analysis of tumor CD8-positive T cells by immunohistochemical (IHC) staining. The right panel shows results of analysis of tumor CD8-positive T cells by flow cytometry (FACS).

Fig. 3 is a diagram showing results of evaluation of the action of removal of CD206-positive M2 macrophages on the cytotoxicity of CD8-positive T cells (evaluation by qPCR of genes associated with the cytotoxicity).

Fig. 4 is a diagram showing results of evaluation of the action of removal of CD206-positive M2 macrophages on the number of fibroblasts locating adjacent to a tumor tissue. The two left panels show results (drawing-substituting photographs) of analysis of tumor CD8-positive T cells and fibroblasts by immunohistochemical (IHC) staining. The two right panels show results of analysis of tumor CD8-positive T cells and fibroblasts.

Fig. 5 is a diagram showing results of evaluation of the action on an activity of fibroblasts locating adjacent to a tumor tissue, which activity inhibits infiltration of CD8-positive T cells into the tumor (evaluation by qPCR of genes associated with infiltration of CD8-positive T cells).

Fig. 6 is a diagram showing results of flow cytometric detection of CD206-positive M2 macrophages by anti-CD206 antibodies to be used in the present invention.

Fig. 7 is a diagram showing results of evaluation of the CDC activities of anti-CD206 antibodies to be used in the present invention, against CD206-positive cells.

Fig. 8 is a diagram showing results of evaluation of the ADCC activities of anti-CD206 antibodies to be used in the present invention, against CD206-positive cells.

Fig. 9 is a diagram showing results of evaluation (qPCR) of the cytotoxic actions of anti-CD206 antibodies to be used in the present invention, against CD206-positive M2 macrophages in biological tissues.

Fig. 10 is a diagram showing results of evaluation (FACS) of the cytotoxic actions of anti-CD206 antibodies to be used in the present invention, against CD206-positive M2 macrophages in biological tissues.

DESCRIPTION OF THE EMBODIMENTS

Embodiments of the present invention are described below. However, the invention is not limited to the following preferred embodiments, and may be arbitrarily modified within the scope of the invention. In the present description, when a numerical range is expressed as "lower limit to upper limit", the upper limit may be either "... or less" or "less than ...", and the lower limit may be either "... or more" or "more than ...".

<Pharmaceutical Composition for Prevention or Treatment of Cancer>

One aspect of the present invention relates to a pharmaceutical composition for the prevention or treatment of a cancer, comprising, as an effective component, a substance having an ability to kill CD206-positive M2 macrophages (which composition may be hereinafter referred to as "pharmaceutical composition for the prevention or treatment of a cancer of the present invention").

As described above, in the study of a novel pharmaceutical composition for the prevention or treatment of a cancer, the present inventors revealed that CD206-positive M2 macrophages contribute positively to the development/progression of a cancer in an in vivo model. It was also found that removal of CD206-positive M2 macrophages enables suppression of occurrence of a tumor, and enables reduction of the size of a tumor. The pharmaceutical composition for the prevention or treatment of a cancer of the present invention was invented based on such discoveries.

The present inventors also revealed that removal of CD206-positive M2 macrophages enables promotion of anti-tumor immune responses such as 1) an increase in the number of CD8-positive T cells; 2) enhancement of the cytotoxicity of CD8-positive T cells; 3) reduction of fibroblasts having a cancer-protective function (in particular, reduction of fibroblasts locating adjacent to cancer cells and hence preventing infiltration of cytotoxic T cells into the cancer tissue, which reduction promotes migration of cytotoxic T cells into the cancer tissue); and 4) reduction of the cancer-protective function of fibroblasts. Such a method is thought to be advantageous in cancer treatment. More specifically, as described later, the present invention provides 1) means for controlling the number and cytotoxicity of CD8-positive T cells that attack a tumor; and 2) means for controlling the number and properties of fibroblasts that inhibit infiltration of CD8-positive T cells into a tumor.

<<Substance Having Capacity to Kill CD206-Positive M2 Macrophages>>

The substance having an ability to kill CD206-positive M2 macrophages used in the present invention is not limited as long as the substance has an ability to act on CD206-positive M2 macrophages to kill (or damage) the CD206-positive M2 macrophages, and removes the CD206-positive M2 macrophages (the removal means not only complete removal, but also partial removal resulting in reduction of the number). For example, the substance may be a substance that directly acts on CD206-positive M2 macrophages to kill (or damage) the CD206-positive M2 macrophages, or a substance that indirectly acts on CD206-positive M2 macrophages to kill (or damage) the CD206-positive M2 macrophages. The substance having an ability to kill CD206-positive M2 macrophages may be either a single substance or a combination of two or more substances.

Specific examples of the substance having an ability to kill CD206-positive M2 macrophages used in the present invention include: low molecular weight compounds; anti-CD206 antibodies, functionally modified antibodies thereof, conjugated antibodies thereof, or functional fragments thereof; and siRNAs (short interfering RNAs), shRNAs (short hairpin RNAs), or antisense oligonucleotides. Among these substances, anti-CD206 antibodies, functionally modified antibodies thereof, conjugated antibodies thereof, or functional fragments thereof are preferred. Anti-CD206 antibodies or functional fragments thereof are more preferred. Anti-CD206 antibodies are especially preferred.

<<Anti-CD206 Antibody>>

The anti-CD206 antibody used in the present invention is not limited as long as it is an antibody capable of binding to CD206 on CD206-positive M2 macrophages to kill the CD206-positive M2 macrophages. The action to kill CD206-positive M2 macrophages may be one or more selected from the CDC activity and the ADCC activity. The anti-CD206 antibody used in the present invention may be either a polyclonal antibody or a monoclonal antibody. The antibody is preferably a monoclonal antibody in the present invention. The anti-CD206 antibody used in the present invention may be either a CD206-monospecific antibody, or a multispecific antibody that recognizes a plurality of antigens including CD206 and another antigen. The antibody is preferably a CD206-monospecific antibody.

The anti-CD206 antibody used in the present invention may be an antibody produced based on a conventional method used in the field of molecular biology. Examples of such an anti-CD206 antibody include: a polyclonal antibody or monoclonal antibody obtained by immunizing a mammal such as a mouse using CD206 protein or a partial fragment thereof (such as the epitope fragment) as an antigen; a chimeric antibody or a humanized antibody produced using a genetic recombination technique; and a human antibody produced using a human-antibody-producing transgenic animal or the like. In cases where the antibody of the present invention is administered as a pharmaceutical to a human, the antibody is preferably a humanized antibody or a human antibody from the viewpoint of side effects. The anti-CD206 antibody used in the present invention can be produced also by the later-described ISAAC method.

<<Anti-CD206 Antibody or Functional Fragment Thereof>>

Specific examples of the anti-CD206 antibody used in the present invention include the later-described (a) to (k), and (A) to (K).

The anti-CD206 antibody used in the present invention can be obtained, for example, by administering a particular antigen to an animal that produces antibodies against the antigen, to immunize the animal, and then recovering antibody-producing cells, followed by performing screening by the ISAAC (ImmunoSpot Array Assay on a Chip) method.

The ISAAC method is a method of rapid and comprehensive screening for antigen-specific antibody-secreting cells (ASCs) using a microwell array chip. This method uses a microwell array chip comprising a large number of wells each having a size at which a single antibody-producing cell can be placed (about 10 to 15 μm in diameter), wherein the chip surface around the wells is coated with an anti-immunoglobulin antibody (or the antigen for which antibodies are to be obtained). From the animal that produces antibodies against the particular antigen, spleen lymphocytes containing antibody-producing cells are collected, and the collected cells are plated one by one on the wells of the micro well array chip. The chip is immersed in a medium, and culture is performed under conditions where secreted antibodies disperse from the wells to the coating layer. By this, each antibody secreted from the cell that produces and secretes the antibody is allowed to bind to the anti-immunoglobulin antibody (or antigen) around the well in which the cell is stored. Subsequently, the antigen (or an antibody against the antigen), after labeling with a fluorescent substance, is added to the microwell array chip. The labeled antigen (or antibody against the antigen) binds to the anti-immunoglobulin antibody (or antigen) around the wells. Based on signals emitted from the labeled substance, wells containing cells that secrete antigen-specific antibodies can be identified. These cells are collected using a microcapillary under a fluorescence microscope. After extracting DNA from the cells, cDNAs of the antibodies are amplified by RT-PCR or the like, followed by cloning of antibody genes. Using these genes, desired antibodies can be prepared as recombinant antibodies.

CD206 is a C-type lectin receptor having a single-pass transmembrane structure, which is also known as a mannose receptor. CD206 is a surface antigen specific to M2 macrophages.

In the preparation of the anti-CD206 antibodies used in the present invention, for example, lymphocytes are collected from the spleen of a CD206-deficient mouse to which a CD206 antigen has been administered, and, by the ISAAC described above, cDNAs of mouse antibodies against CD206 can be obtained. Mouse anti-human CD206 monoclonal antibodies can then be prepared as recombinant antibodies.

The ISAAC method can be carried out according to descriptions in JP 2009-34047 A; JP 2014-73100 A; JP 2014-162772 A; Aishun Jin et al., Nature Medicine, Vol. 15, No. 9, September 2009, pp. 1088-1093; and Aishun Jin et al., Nature Protocols, Vol. 6, No. 5, 2011, pp. 668-676.

As anti-CD206 antibodies used in the present invention, the following 11 kinds of mouse anti-human anti-CD206 monoclonal antibodies were obtained by the ISAAC method: Nos. 10, 27, 35, 40, 44, 47, 54, 59, 72, 73, and 83.

Monoclonal antibodies having the particular sequences described above can be obtained also by using a technique normally used in the art such as chemical synthesis or genetic recombination based on the sequence information.

The anti-CD206 antibodies used in the present invention include not only the monoclonal antibodies described above, but also genetically modified antibodies prepared by artificial modification for the purpose of reducing heterologous antigenicity against humans, such as chimeric antibodies, humanized antibodies, and human antibodies. These antibodies can be produced using known methods. In the present description, the term "antibodies of Nos. 10, 27, 35, 40, 44, 47, 54, 59, 72, 73, and 83" includes chimeric antibodies and humanized antibodies prepared based on the sequences of those antibodies, unless such understanding causes inconsistency.

The anti-CD206 antibodies used in the present invention also include shuffled antibodies containing the H chain or L chain of any of the antibodies of No, 10, 27, 35, 40, 44, 47, 54, 59, 72, 73, and 83.

Each anti-CD206 antibody used in the present invention can be produced by inserting a DNA encoding the heavy chain of the antibody and a DNA encoding the light chain of the antibody into an expression vector(s), transforming host cells using the vector(s), and then culturing the host cells to allow production of the antibody. In this process, the DNA encoding the heavy chain and the DNA encoding the light chain may be inserted into the same expression vector, and the host cells may be transformed using the vector. Alternatively, the DNA encoding the heavy chain and the DNA encoding the light chain may be inserted into separate vectors, and the host cells may be transformed using the two vectors. In this process, DNAs encoding the heavy-chain variable region and the light-chain variable region may be inserted into a vector(s) to which DNAs encoding the heavy-chain constant region and the light-chain constant region of a particular isotype have been preliminarily inserted. The vector(s) may also contain a DNA encoding a signal peptide that promotes secretion of the antibody from the host cells. In this case, the DNA encoding the signal peptide is linked in-frame to the DNA encoding the antibody. Since the signal peptide is removed upon the production of the antibody, the antibody can be obtained as a mature protein.

For each of the 11 kinds of anti-CD206 monoclonal antibodies, the DNA sequence and the amino acid sequence of the heavy-chain variable region, the DNA sequence and the amino acid sequence of the light-chain variable region, the amino acid sequence of each of the CDRs (complementarity determining regions) of the heavy-chain variable region (HCDR1, HCDR2, and HCDR3), and the sequence or SEQ ID NO. of the amino acid sequence of each of the CDRs (complementarity determining regions) of the light-chain variable region (LCDR1, LCDR2, and LCDR3), are described below.

(Antibody No. 10)

An antibody that specifically binds to CD206, or a functional fragment thereof, wherein the antibody is the following antibody:

(a) an antibody containing:

heavy-chain HCDR1 having the amino acid sequence of SEQ ID NO:27;
heavy-chain HCDR2 having the amino acid sequence of SEQ ID NO:28;
heavy-chain HCDR3 having the amino acid sequence of SEQ ID NO:29;
light-chain LCDR1 having the amino acid sequence of SEQ ID NO:30;
light-chain LCDR2 having the amino acid sequence of YTS; and
light-chain LCDR3 having the amino acid sequence of SEQ ID NO:31.

An antibody that specifically binds to CD206, or a functional fragment thereof, wherein the antibody is the following antibody:

(A) an antibody containing:

a heavy-chain variable region having the amino acid sequence of SEQ ID NO:1, or a heavy-chain variable region having an amino acid sequence with a sequence identity of not less than 90% to the amino acid sequence and having a CD206-binding activity; and
a light-chain variable region having the amino acid sequence of SEQ ID NO:2, or a light-chain variable region having an amino acid sequence with a sequence identity of not less than 90% to the amino acid sequence and having a CD206-binding activity.

(Antibody No. 27)

An antibody that specifically binds to CD206, or a functional fragment thereof, wherein the antibody is the following antibody:
(b) an antibody containing:

heavy-chain HCDR1 having the amino acid sequence of SEQ ID NO:37;
heavy-chain HCDR2 having the amino acid sequence of SEQ ID NO:38;
heavy-chain HCDR3 having the amino acid sequence of SEQ ID NO:39;
light-chain LCDR1 having the amino acid sequence of SEQ ID NO:40;
light-chain LCDR2 having the amino acid sequence of LMS; and
light-chain LCDR3 having the amino acid sequence of SEQ ID NO:41.

An antibody that specifically binds to CD206, or a functional fragment thereof, wherein the antibody is the following antibody: (B) an antibody containing:

a heavy-chain variable region having the amino acid sequence of SEQ ID NO:5, or a heavy-chain variable region having an amino acid sequence with a sequence identity of not less than 90% to the amino acid sequence and having a CD206-binding activity; and
a light-chain variable region having the amino acid sequence of SEQ ID NO:6, or a light-chain variable region having an amino acid sequence with a sequence identity of not less than 90% to the amino acid sequence and having a CD206-binding activity.

(Antibody No. 35)

An antibody that specifically binds to CD206, or a functional fragment thereof, wherein the antibody is the following antibody:
(c) an antibody containing:

heavy-chain HCDR1 having the amino acid sequence of SEQ ID NO:42;
heavy-chain HCDR2 having the amino acid sequence of SEQ ID NO:43;
heavy-chain HCDR3 having the amino acid sequence of SEQ ID NO:44;
light-chain LCDR1 having the amino acid sequence of SEQ ID NO:45;
light-chain LCDR2 having the amino acid sequence of RAS; and
light-chain LCDR3 having the amino acid sequence of SEQ ID NO:46.

An antibody that specifically binds to CD206, or a functional fragment thereof, wherein the antibody is the following antibody:
(C) an antibody containing:

a heavy-chain variable region having the amino acid sequence of SEQ ID NO:7, or a heavy-chain variable region having an amino acid sequence with a sequence identity of not less than 90% to the amino acid sequence and having a CD206-binding activity; and
a light-chain variable region having the amino acid sequence of SEQ ID NO:8, or a light-chain variable region having an amino acid sequence with a sequence identity of not less than 90% to the amino acid sequence and having a CD206-binding activity.

(Antibody No. 40)

An antibody that specifically binds to CD206, or a functional fragment thereof, wherein the antibody is the following antibody:
(d) an antibody containing:

heavy-chain HCDR1 having the amino acid sequence of SEQ ID NO:47;
heavy-chain HCDR2 having the amino acid sequence of SEQ ID NO:48;
heavy-chain HCDR3 having the amino acid sequence of SEQ ID NO:49;
light-chain LCDR1 having the amino acid sequence of SEQ ID NO:50;
light-chain LCDR2 having the amino acid sequence of FAS; and

light-chain LCDR3 having the amino acid sequence of SEQ ID NO:51.

An antibody that specifically binds to CD206, or a functional fragment thereof, wherein the antibody is the following antibody:
(D) an antibody containing:

a heavy-chain variable region having the amino acid sequence of SEQ ID NO:9, or a heavy-chain variable region having an amino acid sequence with a sequence identity of not less than 90% to the amino acid sequence and having a CD206-binding activity; and
a light-chain variable region having the amino acid sequence of SEQ ID NO:10, or a light-chain variable region having an amino acid sequence with a sequence identity of not less than 90% to the amino acid sequence and having a CD206-binding activity.

(Antibody No. 44)

An antibody that specifically binds to CD206, or a functional fragment thereof, wherein the antibody is the following antibody:
(e) an antibody containing:

heavy-chain HCDR1 having the amino acid sequence of SEQ ID NO:52;
heavy-chain HCDR2 having the amino acid sequence of SEQ ID NO:53;
heavy-chain HCDR3 having the amino acid sequence of SEQ ID NO:54;
light-chain LCDR1 having the amino acid sequence of SEQ ID NO:55;
light-chain LCDR2 having the amino acid sequence of AAT; and
light-chain LCDR3 having the amino acid sequence of SEQ ID NO:56.

An antibody that specifically binds to CD206, or a functional fragment thereof, wherein the antibody is the following antibody:
(E) an antibody containing:

a heavy-chain variable region having the amino acid sequence of SEQ ID NO:11, or a heavy-chain variable region having an amino acid sequence with a sequence identity of not less than 90% to the amino acid sequence and having a CD206-binding activity; and
a light-chain variable region having the amino acid sequence of SEQ ID NO:12, or a light-chain variable region having an amino acid sequence with a sequence identity of not less than 90% to the amino acid sequence and having a CD206-binding activity.

(Antibody No. 47)

An antibody that specifically binds to CD206, or a functional fragment thereof, wherein the antibody is the following antibody:
(f) an antibody containing:

heavy-chain HCDR1 having the amino acid sequence of SEQ ID NO:57;
heavy-chain HCDR2 having the amino acid sequence of SEQ ID NO:58;
heavy-chain HCDR3 having the amino acid sequence of SEQ ID NO:59;
light-chain LCDR1 having the amino acid sequence of SEQ ID NO:60;
light-chain LCDR2 having the amino acid sequence of YTS; and
light-chain LCDR3 having the amino acid sequence of SEQ ID NO:61.

An antibody that specifically binds to CD206, or a functional fragment thereof, wherein the antibody is the following antibody:
(F) an antibody containing:

a heavy-chain variable region having the amino acid sequence of SEQ ID NO:13, or a heavy-chain variable region having an amino acid sequence with a sequence identity of not less than 90% to the amino acid sequence and having a CD206-binding activity; and
a light-chain variable region having the amino acid sequence of SEQ ID NO:14, or a light-chain variable region having an amino acid sequence with a sequence identity of not less than 90% to the amino acid sequence and having a CD206-binding activity.

(Antibody No. 54)

An antibody that specifically binds to CD206, or a functional fragment thereof, wherein the antibody is the following antibody:
(g) an antibody containing:

heavy-chain HCDR1 having the amino acid sequence of SEQ ID NO:62;
heavy-chain HCDR2 having the amino acid sequence of SEQ ID NO:63;
heavy-chain HCDR3 having the amino acid sequence of SEQ ID NO:64;
light-chain LCDR1 having the amino acid sequence of SEQ ID NO:65;
light-chain LCDR2 having the amino acid sequence of KAS; and
light-chain LCDR3 having the amino acid sequence of SEQ ID NO:66.

An antibody that specifically binds to CD206, or a functional fragment thereof, wherein the antibody is the following antibody:
(G) an antibody containing:

a heavy-chain variable region having the amino acid sequence of SEQ ID NO:15, or a heavy-chain variable region having an amino acid sequence with a sequence identity of not less than 90% to the amino acid sequence and having a CD206-binding activity; and
a light-chain variable region having the amino acid sequence of SEQ ID NO:16, or a light-chain variable region having an amino acid sequence with a sequence identity of not less than 90% to the amino acid sequence and having a CD206-binding activity.

(Antibody No. 59)

An antibody that specifically binds to CD206, or a functional fragment thereof, wherein the antibody is the following antibody:
(h) an antibody containing:

heavy-chain HCDR1 having the amino acid sequence of SEQ ID NO:67;
heavy-chain HCDR2 having the amino acid sequence of SEQ ID NO:68;
heavy-chain HCDR3 having the amino acid sequence of SEQ ID NO:69;
light-chain LCDR1 having the amino acid sequence of SEQ ID NO:70;
light-chain LCDR2 having the amino acid sequence of YTS; and
light-chain LCDR3 having the amino acid sequence of SEQ ID NO:71.

An antibody that specifically binds to CD206, or a functional fragment thereof, wherein the antibody is the following antibody:
(H) an antibody containing:

a heavy-chain variable region having the amino acid sequence of SEQ ID NO:17, or a heavy-chain variable region having an amino acid sequence with a sequence identity of not less than 90% to the amino acid sequence and having a CD206-binding activity; and
a light-chain variable region having the amino acid sequence of SEQ ID NO:18, or a light-chain variable region having an amino acid sequence with a sequence identity of not less than 90% to the amino acid sequence and having a CD206-binding activity.

(Antibody No. 72)

An antibody that specifically binds to CD206, or a functional fragment thereof, wherein the antibody is the following antibody:
(i) an antibody containing:

heavy-chain HCDR1 having the amino acid sequence of SEQ ID NO:77;

heavy-chain HCDR2 having the amino acid sequence of SEQ ID NO:78;
heavy-chain HCDR3 having the amino acid sequence of SEQ ID NO:79;
light-chain LCDR1 having the amino acid sequence of SEQ ID NO:80;
light-chain LCDR2 having the amino acid sequence of ATS; and
light-chain LCDR3 having the amino acid sequence of SEQ ID NO:81.

An antibody that specifically binds to CD206, or a functional fragment thereof, wherein the antibody is the following antibody:
(I) an antibody containing:

a heavy-chain variable region having the amino acid sequence of SEQ ID NO:21, or a heavy-chain variable region having an amino acid sequence with a sequence identity of not less than 90% to the amino acid sequence and having a CD206-binding activity; and
a light-chain variable region having the amino acid sequence of SEQ ID NO:22, or a light-chain variable region having an amino acid sequence with a sequence identity of not less than 90% to the amino acid sequence and having a CD206-binding activity.

(Antibody No. 73)

An antibody that specifically binds to CD206, or a functional fragment thereof, wherein the antibody is the following antibody:
(j) an antibody containing:

heavy-chain HCDR1 having the amino acid sequence of SEQ ID NO:82;
heavy-chain HCDR2 having the amino acid sequence of SEQ ID NO:83;
heavy-chain HCDR3 having the amino acid sequence of SEQ ID NO:84;
light-chain LCDR1 having the amino acid sequence of SEQ ID NO:85;
light-chain LCDR2 having the amino acid sequence of RAN; and
light-chain LCDR3 having the amino acid sequence of SEQ ID NO:86.

An antibody that specifically binds to CD206, or a functional fragment thereof, wherein the antibody is the following antibody:
(J) an antibody containing:

a heavy-chain variable region having the amino acid sequence of SEQ ID NO:25, or a heavy-chain variable region having an amino acid sequence with a sequence identity of not less than 90% to the amino acid sequence and having a CD206-binding activity; and
a light-chain variable region having the amino acid sequence of SEQ ID NO:26, or a light-chain variable region having an amino acid sequence with a sequence identity of not less than 90% to the amino acid sequence and having a CD206-binding activity.

(Antibody No. 83)

An antibody that specifically binds to CD206, or a functional fragment thereof, wherein the antibody is the following antibody:
(k) an antibody containing:

heavy-chain HCDR1 having the amino acid sequence of SEQ ID NO:87;
heavy-chain HCDR2 having the amino acid sequence of SEQ ID NO:88;
heavy-chain HCDR3 having the amino acid sequence of SEQ ID NO:89;
light-chain LCDR1 having the amino acid sequence of SEQ ID NO:90;
light-chain LCDR2 having the amino acid sequence of YTS; and
light-chain LCDR3 having the amino acid sequence of SEQ ID NO:91.

An antibody that specifically binds to CD206, or a functional fragment thereof, wherein the antibody is the following antibody:
(K) an antibody containing:

a heavy-chain variable region having the amino acid sequence of SEQ ID NO:25, or a heavy-chain variable region having an amino acid sequence with a sequence identity of not less than 90% to the amino acid sequence and having a CD206-binding activity; and

a light-chain variable region having the amino acid sequence of SEQ ID NO:26, or a light-chain variable region having an amino acid sequence with a sequence identity of not less than 90% to the amino acid sequence and having a CD206-binding activity.

In the 11 kinds of anti-CD206 monoclonal antibodies, examples of the heavy-chain variable region include not only a heavy-chain variable region having the amino acid sequence of each SEQ ID NO., but also a heavy-chain variable region composed of a protein having the same amino acid sequence except that one or several, for example, 1 to 10, 1 to 5, 1 to 3, 1 to 2, or one, amino acid(s) is/are deleted, substituted, and/or added, which protein has the activity of the heavy-chain variable region of the antibody, in other words, the binding activity to human CD206. Similarly, examples of the light-chain variable region include not only a light-chain variable region having the amino acid sequence of each SEQ ID NO., but also a light-chain variable region composed of a protein having the same amino acid sequence except that one or several, for example, 1 to 10, 1 to 5, 1 to 3, 1 to 2, or one, amino acid(s) is/are deleted, substituted, and/or added, which protein has the activity of the light-chain variable region, in other words, the binding activity to human CD206.

Although such a substitution(s), deletion(s), and/or addition(s) may be introduced to the CDRs, those are preferably introduced to a region(s) other than the CDRs. From the viewpoint of maintaining the characteristics of the present invention, the amino acid substitution(s) is/are preferably a conservative substitution(s). The conservative substitution herein means that an amino acid residue is substituted by another chemically similar amino acid residue such that no substantial change occurs in the activity of the peptide. Examples of such a substitution include cases where a certain hydrophobic residue is substituted by another hydrophobic residue, and cases where a certain polar residue is substituted by another polar residue. Examples of functionally similar amino acids among which such substitutions are possible include nonpolar (hydrophobic) amino acids such as alanine, valine, isoleucine, leucine, proline, tryptophan, phenylalanine, and methionine. Examples of polar (neutral) amino acids include glycine, serine, threonine, tyrosine, glutamine, asparagine, and cysteine. Examples of amino acids having a positive charge (basic amino acids) include arginine, histidine, and lysine. Examples of amino acids having a negative charge (acidic amino acids) include aspartic acid and glutamic acid.

Examples of such an amino acid sequence which is the same except that one or several amino acid(s) is/are deleted, substituted, and/or added include an amino acid sequence having a sequence identity of, for example, not less than 85%, not less than 90%, not less than 95%, not less than 97%, not less than 98%, or not less than 99%, to the amino acid sequence of each SEQ ID NO. as calculated using BLAST (Basic Local Alignment Search Tool at the National Center for Biological Information) or the like (with, for example, the default parameters, in other words, the initially set parameters).

A protein having such an amino acid sequence which is the same except that one or several amino acid(s) is/are deleted, substituted, and/or added is substantially identical to a protein having the amino acid sequence of the corresponding SEQ ID NO.

**[0058]** Examples of the DNA encoding the amino acid sequence of each SEQ ID NO. described above include a DNA having a base sequence with a sequence identity of, for example, not less than 85%, not less than 90%, not less than 95%, not less than 97%, not less than 98%, or not less than 99%, to the DNA sequence as calculated using BLAST (Basic Local Alignment Search Tool at the National Center for Biological Information) or the like (with, for example, the default parameters, in other words, the initially set parameters), which DNA encodes a protein having the activity of the heavy-chain variable region or the light-chain variable region of the antibody, in other words, the binding activity to human CD206. Such a DNA is also included in the DNA encoding the heavy-chain variable region or the light-chain variable region of the anti-CD206 antibody.

Examples of the DNA encoding the heavy-chain variable region or the light-chain variable region of the anti-CD206 antibody also include a DNA which is capable of hybridizing, under stringent conditions, with the DNA having the sequence complementary to a DNA encoding the amino acid sequence of each SEQ ID NO., and which encodes a protein having the activity of the heavy-chain variable region or the light-chain variable region of the antibody, in other words, the binding activity to human CD206. Here, those skilled in the art can appropriately determine the stringent conditions.

The human anti-human CD206 monoclonal antibody that specifically binds to human CD206, containing the heavy-chain variable region and the light-chain variable region, is constituted by the above-described heavy-chain variable region and a heavy-chain constant region, and the above-described light-chain variable region and a light-chain constant region. The heavy-chain constant region is constituted by the three domains $C_H1$, $C_H2$, and $C_H3$. The heavy-chain constant region may be the IgG1, IgG2, IgG3, IgG4, IgA, IgE, IgM, or IgD constant region, and is most preferably the IgG1 or IgG4 constant region. The light-chain constant region is constituted by the one domain $C_L$. The light-chain constant region is the $\kappa$ or $\lambda$ constant region.

The DNA encoding the antibody is obtained as a DNA encoding the heavy chain and a DNA encoding the light chain, by linking the DNA encoding the heavy-chain variable region to the DNA encoding the heavy-chain constant region, and also linking the DNA encoding the light-chain variable region to the DNA encoding the light-chain constant region.

The anti-CD206 antibody used in the invention also encompasses functional fragments of the antibody or modified products thereof. For example, the functional fragments of the antibody are fragments of the antibody, which fragments are capable of specifically binding to the antigen. Examples of the functional fragments include Fab, $F(ab')_2$, Fv, Fab/c, which contains one Fab and the complete Fc, and a single-chain Fv (scFv), which contains the H-chain and L-chain Fv linked via an appropriate linker. The polynucleotide encompasses both DNA and RNA.

The anti-CD206 antibody binds to CD206, preferably the extracellular domain of CD206 present on the cell membrane surface of M2 macrophages, and has a cytotoxic action against CD206-positive M2 macrophages. More specifically, the cytotoxic action may be one or more selected from the CDC activity and the ADCC activity. Thus, the anti-CD206 antibody has a cytotoxic action against CD206-positive M2 macrophages, and is capable of removing CD206-positive M2 macrophages from biological tissues and the like. The antibody has, for example, an anticancer action, an action to kill fibroblasts locating adjacent to a tumor tissue, an action to promote infiltration of cytotoxic T cells into a tumor tissue, an action to suppress the expression of a gene(s) in fibroblasts, which gene(s) suppress(es) infiltration of cytotoxic T cells into a tumor, an action to promote the growth of cytotoxic T cells, an action to enhance the cytotoxic activity of cytotoxic T cells, and an action to enhance the expression of a gene(s) associated with the cytotoxicity of cytotoxic T cells. The antibody can therefore be used as an effective component of a pharmaceutical composition for the prevention or treatment of a cancer, a composition for killing fibroblasts locating adjacent to a tumor tissue, a composition for promoting infiltration of cytotoxic T cells into a tumor tissue, a composition for suppressing the expression of a gene(s) in fibroblasts, which gene(s) suppress(es) infiltration of cytotoxic T cells into a tumor, a composition for promoting the growth of cytotoxic T cells, a composition for enhancing the cytotoxic activity of cytotoxic T cells, and a composition for enhancing the expression of a gene(s) associated with the cytotoxicity of cytotoxic T cells.

Any of the 11 kinds of mouse anti-human CD206 monoclonal antibodies can be used as a substance having an ability to kill CD206-positive M2 macrophages, which substance is the effective component of the pharmaceutical composition for the prevention or treatment of a cancer, the composition for killing fibroblasts locating adjacent to a tumor tissue, the composition for promoting infiltration of cytotoxic T cells into a tumor tissue, the composition for suppressing the expression of a gene(s) in fibroblasts, which gene(s) suppress(es) infiltration of cytotoxic T cells into a tumor, the composition for promoting the growth of cytotoxic T cells, the composition for enhancing the cytotoxic activity of cytotoxic T cells, and the composition for enhancing the expression of a gene(s) associated with the cytotoxicity of cytotoxic T cells, of the present invention. In particular, the antibody Nos. 10, 35, 54, and 83 are preferred since they strongly bind to CD206, although the CD206 antibody is not limited thereto. The antibody may be either a single antibody or an arbitrary combination of two or more antibodies.

The pharmaceutical composition for the prevention or treatment of a cancer of the present invention can be produced based on a conventional method in the field of pharmaceutical preparations. As long as the effect is not impaired, the pharmaceutical composition for the prevention or treatment of a cancer of the present invention may be used in combination with an effective component other than the effective component that is the substance having an ability to kill CD206-positive M2 macrophages. The pharmaceutical composition for the prevention or treatment of a cancer of the present invention may contain a carrier, a diluent, or an excipient normally used in the field of pharmaceutical preparations, in addition to the effective component that is the substance having an ability to kill CD206-positive M2 macrophages. Examples of a carrier or an excipient for tablets that may be used include lactose and magnesium stearate. Examples of an aqueous liquid that may be used for injection include: physiological saline; PBS; and isotonic solutions containing glucose or another auxiliary composition; which may be used in combination with an appropriate solubilizer, such as an alcohol, a polyalcohol including propylene glycol, or a nonionic surfactant. Examples of an oily liquid that may be used include sesame oil and soybean oil, which may be used in combination with a solubilizer such as benzyl benzoate or benzyl alcohol. The pharmaceutical composition for the prevention or treatment of a cancer of the present invention may be administered in various forms, and examples of the dosage forms include oral administration using tablets, capsules, granules, powders, syrups, or the like, and parenteral administration using injection solutions, drops, suppositories, or the like.

The dose varies depending on the symptom, age, body weight, and the like. In oral administration, the dose is usually, for example, about 0.01 mg to 1000 mg per day per adult, and this dose may be administered as a single dose or in several divided doses. In cases of parenteral administration, a dose of, for example, about 0.01 mg to 1000 mg may be administered each time by subcutaneous injection, intramuscular injection, or intravenous injection.

Examples of the cancer that can be prevented or treated include, but are not limited to, lung cancer, gastric cancer, liver cancer, esophageal cancer, pancreatic cancer, colorectal cancer, biliary tract cancer, renal cancer, bladder cancer, uterine cancer, ovarian cancer, breast cancer, prostate cancer, testicular cancer, skin cancer, bone tumor, osteosarcoma, soft tissue tumor, pharyngeal cancer, head and neck cancer, and childhood cancer.

The subject (preferably a mammal, especially a human) for whom the cancer is to be prevented or treated in the present invention is a subject diagnosed as having a high risk of development of the cancer or a subject who developed the cancer. The pharmaceutical composition for the prevention or treatment of a cancer of the present invention may be administered to such a subject.

<Composition for Killing Fibroblasts Locating adjacent to Tumor Tissue>

Another aspect of the present invention relates to a composition for killing fibroblasts locating adjacent to a tumor tissue, the composition comprising, as an effective component, a substance having an ability to kill CD206-positive M2 macrophages (which composition may be hereinafter referred to as "composition for killing fibroblasts locating adjacent to a tumor tissue of the present invention").

In the present description, the fibroblasts locating adjacent to a tumor tissue are, in other words, for example, fibroblasts localized in the tumor microenvironment, or fibroblasts localized in the tumor microenvironment and capable of giving a protective function to a tumor. The fibroblasts locating adjacent to a tumor tissue may also be fibroblasts present such that the fibroblasts surround the tumor tissue.

As described above, the present inventors revealed that removal of CD206-positive M2 macrophages enables reduction of fibroblasts which are localized in the vicinity of a tumor tissue and which have a cancer-protective function. It is thought that, by the reduction of the fibroblasts having a cancer-protective function, anti-tumor immune responses are enhanced. In particular, the removal of the fibroblasts locating adjacent to a tumor tissue allows a composition/a substance having an anti-tumor action, or cells, to easily infiltrate the tumor tissue, thereby promoting killing of the tumor.

The optional components, the dose, the target cancer, the subject for whom the cancer is to be prevented or treated, and the like described in the <Pharmaceutical Composition for Prevention or Treatment of Cancer> section may be applied also to the composition for killing fibroblasts locating adjacent to a tumor tissue of the present invention. The composition for killing fibroblasts locating adjacent to a tumor tissue of the present invention can be used also as a reagent. The production, use, and the like of the reagent may be carried out based on conventional methods in the field of molecular biology.

<Composition for Promoting Infiltration of Cytotoxic T Cells into Tumor Tissue>

Another aspect of the present invention relates to a composition for promoting infiltration of cytotoxic T cells into a tumor tissue, the composition comprising, as an effective component, a substance having an ability to kill CD206-positive M2 macrophages (which composition may be hereinafter referred to as "composition for promoting infiltration of cytotoxic T cells into a tumor tissue of the present invention").

In the present description, the term "cytotoxic T lymphocyte" or "cytotoxic T cell" (CTL) is used interchangeably with the term "CD8-positive T cell", and means a subgroup of T lymphocytes capable of recognizing non-self cells (such as tumor/cancer cells and virus-infected cells) to induce death of such cells.

As described above, the present inventors revealed that removal of CD206-positive M2 macrophages enables reduction of the infiltration-inhibiting function of fibroblasts localized in the vicinity of a tumor tissue, which function inhibits infiltration of cytotoxic T cells into a tumor. It is thought that, by reducing the infiltration-inhibiting function of the fibroblasts to reduce the cancer-protective function, anti-tumor immune responses are enhanced. In one aspect, the promotion of infiltration of cytotoxic T cells into the tumor tissue involves reduction of fibroblasts locating adjacent to the tumor tissue. It is thought that the reduction of the fibroblasts locating adjacent to the tumor tissue enables easy infiltration (introduction/entry) of cytotoxic T cells into the tumor tissue. In one aspect, the promotion of infiltration of cytotoxic T cells into the tumor tissue involves suppression of the expression of a gene(s) (for example, one or more genes selected from Acta2, CXCL12, and CXCL14) on fibroblasts locating adjacent to the tumor tissue, which gene(s) suppress(es) infiltration of cytotoxic T cells into the tumor. Here, the suppression of the gene(s) can be evaluated based on the expression of the protein encoded by each gene, the amount of the protein secreted, the expression level of each gene (mRNA or cDNA), or the like. Since the composition of the present invention also enables easy introduction of an anticancer drug or the like into the tumor tissue, the present invention can be used also as a cancer treatment aid.

The optional components, the dose, the target cancer, the subject for whom the cancer is to be prevented or treated, and the like described in the <Pharmaceutical Composition for Prevention or Treatment of Cancer> section may be applied also to the composition for promoting infiltration of cytotoxic T cells into a tumor tissue of the present invention. The composition for promoting infiltration of cytotoxic T cells into a tumor tissue of the present invention can be used also as a reagent. The production, use, and the like of the reagent may be carried out based on conventional methods in the field of molecular biology.

<Composition for Suppressing Expression of Gene(s) in Fibroblasts, Which Gene(s) Suppress(es) Infiltration of Cytotoxic T Cells into Tumor>

Another aspect of the present invention relates to a composition for suppressing the expression of one or more genes selected from Acta2, CXCL12, and CXCL14 on fibroblasts locating adjacent to a tumor tissue, the composition comprising, as an effective component, a substance having an ability to kill CD206-positive M2 macrophages (which composition may be hereinafter referred to as "composition for suppressing the expression of a gene(s) in fibroblasts, which gene(s) suppress(es) infiltration of cytotoxic T cells into a tumor, of the present invention").

The present inventors removed CD206-positive M2 macrophages to reveal that promotion of infiltration of cytotoxic T cells into the tumor tissue enables suppression of the expression of a gene(s) (for example, one or more genes selected from Acta2, CXCL12, and CXCL14) on fibroblasts locating adjacent to the tumor tissue, which gene(s) suppress(es) infiltration of cytotoxic T cells into the tumor. The suppression of the expression of the gene(s) is thought to contribute to reduction of the infiltration-inhibiting function in fibroblasts localized in the vicinity of a tumor tissue, which function inhibits infiltration of cytotoxic T cells into a tumor.

The optional components, the dose, the target cancer, the subject for whom the cancer is to be prevented or treated, and the like described in the <Pharmaceutical Composition for Prevention or Treatment of Cancer> section may be applied also to the composition for suppressing the expression of a gene(s) in fibroblasts, which gene(s) suppress(es) infiltration of cytotoxic T cells into a tumor, of the present invention. The composition for suppressing the expression of a gene(s) in fibroblasts, which gene(s) suppress(es) infiltration of cytotoxic T cells into a tumor, of the present invention can be used also as a reagent. The production, use, and the like of the reagent may be carried out based on conventional methods in the field of molecular biology.

<Composition for Promoting Growth of Cytotoxic T Cells>

Another aspect of the present invention relates to a composition for promoting the growth of cytotoxic T cells, the composition comprising, as an effective component, a substance having an ability to kill CD206-positive M2 macrophages (which composition may be hereinafter referred to as "composition for promoting the growth of cytotoxic T cells").

As described above, the present inventors revealed that removal of CD206-positive M2 macrophages enables increasing of the number of CD8-positive T cells that attack a tumor. It is thought that the increase in the number of CD8-positive T cells enhances anti-tumor immune responses. It is also thought that the increase in the number of cytotoxic T cells leads to enhancement of not only the anti-tumor immune responses, but also immune responses based on the cytotoxic activity of the cytotoxic T cells, such as anti-viral immune responses and anti-bacterial immune responses. Thus, the composition for promoting the growth of cytotoxic T cells of the present invention can be used also as a therapeutic composition for a disease for which curing based on the cytotoxic activity of cytotoxic T cells can be expected, or a treatment aid for the disease.

The optional components, the dose, the target cancer, the subject for whom the cancer is to be prevented or treated, and the like described in the <Pharmaceutical Composition for Prevention or Treatment of Cancer> section may be applied also to the composition for promoting the growth of cytotoxic T cells of the present invention. The composition for promoting the growth of cytotoxic T cells of the present invention can be used also as a reagent. The production, use, and the like of the reagent may be carried out based on conventional methods in the field of molecular biology.

<Composition for Enhancing Cytotoxic Activity of Cytotoxic T Cells>

Another aspect of the present invention relates to a composition for enhancing the cytotoxic activity of cytotoxic T cells, the composition comprising, as an effective component, a substance having an ability to kill CD206-positive M2 macrophages (which composition may be hereinafter referred to as "composition for enhancing the cytotoxic activity of cytotoxic T cells of the present invention").

As described above, the present inventors revealed that removal of CD206-positive M2 macrophages enables enhancement of the cytotoxicity of cytotoxic T cells that attack a tumor. It is thought that the enhancement of the cytotoxic activity of cytotoxic T cells enhances anti-tumor immune responses. It is also thought that the enhancement of the cytotoxic activity of cytotoxic T cells leads not only to the anti-tumor immune responses, but also to immune responses based on the cytotoxic activity of the cytotoxic T cells, such as anti-viral immune responses and anti-bacterial immune responses. Thus, the composition for enhancing the cytotoxic activity of cytotoxic T cells of the present invention can be used also as a therapeutic composition for a disease for which curing based on the cytotoxic activity of cytotoxic T cells can be expected, or a treatment aid for the disease. In one aspect, the enhancement of the cytotoxic activity of the cytotoxic T cells involves enhancement of the expression of a gene(s) (for example, one or more genes selected from TNF$\alpha$, IFN$\gamma$, and Gzmb) associated with the cytotoxicity of the cytotoxic T cells. Here, the suppression of the gene(s) can be evaluated based on the expression of the protein encoded by each gene, the amount of the protein secreted, the expression level of each gene (mRNA or cDNA), or the like.

The optional components, the dose, the target tumor, the subject for whom the tumor is to be prevented or treated, and the like described in the <Pharmaceutical Composition for Prevention or Treatment of Cancer> section may be applied also to the composition for enhancing the cytotoxic activity of cytotoxic T cells of the present invention. The composition for enhancing the cytotoxic activity of cytotoxic T cells of the present invention can be used also as a reagent. The production, use, and the like of the reagent may be carried out based on conventional methods in the field of molecular biology.

<Composition for Enhancing Expression of Gene(s) Associated with Cytotoxic Activity of Cytotoxic T Cells >

Another aspect of the present invention relates to a composition for enhancing the expression of one or more genes selected with TNFα, IFNγ, and Gzmb, the composition comprising, as an effective component, a substance having an ability to kill CD206-positive M2 macrophages (which composition may be hereinafter referred to as "composition for enhancing the expression of a gene(s) associated with the cytotoxicity of cytotoxic T cells of the present invention").

As described above, the present inventors revealed that removal of CD206-positive M2 macrophages enables enhancement of the expression of a gene(s) (for example, one or more genes selected from TNFα, IFNγ, and Gzmb) associated with the cytotoxicity of cytotoxic T cells. It is thought that the enhancement of the expression of the gene(s) contributes to enhancement of the cytotoxic activity of cytotoxic T cells that attack a tumor.

The optional components, the dose, the target tumor, the subject for whom the tumor is to be prevented or treated, and the like described in the <Pharmaceutical Composition for Prevention or Treatment of Cancer> section may be applied also to the composition for enhancing the expression of a gene(s) associated with the cytotoxicity of cytotoxic T cells of the present invention. The composition for enhancing the expression of a gene(s) associated with the cytotoxicity of cytotoxic T cells of the present invention can be used also as a reagent. The production, use, and the like of the reagent may be carried out based on conventional methods in the field of molecular biology.

EXAMPLES

The present invention is described below more concretely by way of Examples. However, the present invention is not limited to these Examples as long as the spirit of the present invention is not spoiled.

<Example 1: Study of Action of CD206-Positive M2 Macrophages on Tumor>

Analysis of a tumor in a reporter mouse for CD206-positive M2 macrophages, which mouse develops breast cancer, revealed that CD206-positive M2 macrophages are distributed such that they surround the tumor, and are adjacent to blood vessels. Based on this result, CD206-DTR/PyMT mice (mice which develop breast cancer, and in which CD206-positive macrophages can be reduced by administration of diphtheria toxin) were prepared as follows, and CD206-positive M2 macrophages were removed to study an action of CD206-positive M2 macrophages on a tumor.

(CD206-DTR/PyMT Mice)

PyMT mice (purchased from The Jackson Laboratory), which spontaneously develop breast cancer, were mated with CD206-DTR mice, from which CD206-positive cells can be removed (Nat. Commun. 8, 554 1-15 (2017)), to produce mice which develop breast cancer and from which CD206-positive cells can be removed (CD206-DTR/PyMT mice).

Methods: In the CD206-DTR/PyMT mice, the occurrence, the weight, and the volume of a tumor in cases where CD206-positive macrophages were reduced was evaluated. More specifically, diphtheria toxin was intraperitoneally administered to each of CD206-DTR/PyMT mice and control PyMT mice at a concentration of 3 ng/mouse body weight (g) every three days from the beginning of the experiment (CD206-DTR/PyMT mice, n = 19; PyMT mice, n = 17). The presence or absence of a tumor was investigated by palpation of the mouse mammary glands, and the tumor free rate (the number of mice which developed a tumor / the total number of mice in each group) was calculated using the analysis software JMP15 (SAS Institute), to evaluate the occurrence of a tumor in the cases where CD206-positive macrophages were reduced. Nine weeks after the beginning of the experiment, the CD206-DTR/PyMT mice and the control PyMT mice were euthanized, and the tumor weight and volume in the cases where CD206-positive macrophages were reduced were evaluated. At this time, the total weight of all tumors was measured using a digital scale (A&D Company). In the evaluation of the volume, the sizes of all tumors were measured using a DIGITAL CALIPER DN-100 (Niigataseiki), and the analysis software JMP15 (SAS Institute) was used together with the volume calculation mathematical model of m1 $\times$ m1 $\times$ m2 $\times$ 0.5236 (m1: the shorter axis; m2: the longer axis).

Results: The results are shown in Fig. 1. The reduction of the CD206-positive M2 macrophages resulted in suppression of the occurrence of tumors, and reduction of the tumor weight and the tumor volume. It was thus shown that CD206-positive M2 macrophages contribute positively to the development/progression of a cancer in the in vivo model. It was also demonstrated that, by reduction of CD206-positive M2 macrophages, a tumor-reducing effect can be expected.

<Example 2: Study of Action of CD206-Positive M2 Macrophages on CD8-Positive T Cells>

An action of CD206-positive M2 macrophages in the cancer microenvironment was studied as follows. More specifically, an action of CD206-positive M2 macrophages on CD8-positive T cells was studied.

<2-1: Study of Action on Number of CD8-Positive T Cells>

Methods: CD8-positive T cells in the tumors removed in Example 1 were analyzed by immunohistochemistry (IHC) staining and flow cytometry (FACS). More specifically, staining was carried out using an anti-CD8 antibody (53-6.7 (Biolegend)), a 647-labeled anti-CD206 antibody (MCA2235 (Biorad)), a secondary antibody (Life Technologies), and DAPI (counterstaining). Observation was then carried out under a fluorescence microscope (Leica TCS SP5 (Leica Microsystems)). Further, CD8-positive T cells in the tumors were stained with an APC-labeled anti-CD8 antibody (53-6.7 (Biolegend)), and analyzed with FACSAria (registered trademark) (manufactured by Becton, Dickinson and Company, Japan).

[Analysis of CD8-Positive T Cells]

1. Tumors were collected and homogenized.
2. Treatment with collagenase was carried out for 30 min.
3. Filtration was carried out through a 100-μm filter.
4. Erythrocytes were destroyed using a lysing buffer.
5. Centrifugation was performed at 1500 rpm for 5 min, followed by discarding the supernatant.
6. Cells were resuspended with a buffer.
7. An APC-labeled anti-CD8 antibody was added, followed by incubation for 30 min.
8. Dead cells were removed with 7AAD.
9. CD8-positive T cells were analyzed using FACSAria.

Results: The results are shown in Fig. 2. The reduction of the CD206-positive macrophages resulted in an increase in CD8-positive T cells in the tumors. In general, CD8-positive T cells are cells having cytotoxicity, and attack tumors. The larger the number of those cells, the more the tumor growth suppression can be expected.

<2-2: Study of Action on Cytotoxicity of CD8-Positive T Cells>

Methods: Genes associated with the cytotoxicity of CD8-positive T cells in the tumors removed in Example 1 were investigated by qPCR. For the collection of the CD8-positive T cells from the tumors, the CD8+ T cell isolation kit (Miltenyi Biotec) was used.

[qPCR]

1. mRNA was prepared from CD8-positive T cells of the tumors using the RNeasy kit (Qiagen).
2. cDNA was prepared using the Prime Script (registered trademark) RT master mix (TAKARA) and a Veriti (registered trademark) 96 Well Thermal Cycler (Applied Biosystem).
3. qPCR was carried out with the TB Green Fast qPCR mix (TAKARA) and target gene primers (Invitrogen), using MX3000p (Agilent Technologies).

Results: The results are shown in Fig. 3. The reduction of CD206-positive macrophages resulted in increases in genes (TNFα, IFNγ, and Gzmb) associated with the cytotoxicity of CD8-positive T cells. These changes are thought to be indicative of upregulation of the cytotoxicity of CD8-positive T cells.

<Example 3: Study of Action of CD206-Positive M2 Macrophages on Fibroblasts Locating adjacent to Tumor>

An action of CD206-positive M2 macrophages in the cancer microenvironment was studied as follows. More specifically, an action on fibroblasts locating adjacent to a tumor tissue was studied.

<3-1: Study of Action on Number of Fibroblasts Locating adjacent to Tumor Tissue> (PDGFRα-GFP-CreERT2/CD206-DTR Mice)

PDGFRα-GFP-CreERT2 mice (distributed by a collaborator) were mated with CD206-DTR mice, to produce mice to which breast cancer can be implanted, from which CD206-positive cells can be removed, and in which PDGFRα-positive cells can be traced by GFP (PDGFRα-GFP-CreERT2/CD206-DTR mice).

Methods: each of PDGFRα-GFP-CreERT2/CD206-DTR mice after breast cancer implantation and control PDGFRα-GFP-CreERT2 mice after breast cancer implantation was subjected to intraperitoneal administration of diphtheria toxin at a concentration of 20 ng/mouse body weight (g) every other day from Week 7 after the tumor implantation (PDGFRα-GFP-CreERT2/CD206-DTR mice, n = 3; PDGFRα-GFP-CreERT2 mice, n = 3). Five days after the beginning of administration of diphtheria toxin, the mice were euthanized, and tumors were removed.

Fibroblasts in the removed tumors were analyzed by immunohistochemistry (IHC) staining. More specifically, staining was carried out using an anti-CD8 antibody (53-6.7 (Biolegend)), a 647-labeled anti-CD206 antibody (MCA2235 (Biorad)), a secondary antibody (Life Technologies), and DAPI (counterstaining). Observation was then carried out under a fluorescence microscope (Leica TCS SP5 (Leica Microsystems)). The numbers of CD8-positive T cells and fibroblasts were evaluated by calculating the number of CD8-positive cells and the GFP area index (GFP-positive site area / GFP-positive site area + GFP-negative site area) using the image analysis software ImageJ (NIH) and the statistical analysis software JMP15 (SAS Institute).

Results: The results are shown in Fig. 4. The reduction of the CD206-positive macrophages resulted in an increase in CD8-positive T cells in the tumors, and a decrease in PDGFRα-positive fibroblasts labeled with GFP in the vicinity of the tumors. It is thought that reduction of fibroblasts promotes infiltration of CD8-positive T cells into tumors.

<3-2: Study of Action on Activity of Fibroblasts Locating adjacent to Tumor Tissue, Which Activity Inhibits Infiltration of CD8-Positive T Cells into Tumor>

Methods: For fibroblasts of the tumors removed in Example 1, genes that suppress infiltration of CD8-positive T cells into a tumor were investigated by qPCR.

[Collection of Fibroblasts]

1. Tumors were collected and homogenized.
2. Treatment with collagenase was carried out for 30 min.
3. Filtration was carried out through a 100-μm filter.
4. Erythrocytes were destroyed using a lysing buffer.
5. Centrifugation was performed at 1500 rpm for 5 min, followed by discarding the supernatant.
6. Cells were resuspended with a buffer.
7. PDGFRα-positive fibroblasts were collected using the PDGFRα Micro Bead Kit (Miltenyi Biotec).

[qPCR]

1. mRNA was prepared from CD8-positive T cells of tumors using the RNeasy kit (Qiagen).
2. cDNA was prepared using the Prime Script RT master mix (TAKARA) and a Veriti 96 Well Thermal Cycler (Applied Biosystem).
3. qPCR was carried out with the TB Green Fast qPCR mix (TAKARA) and target gene primers (Invitrogen), using MX3000p (Agilent technologies).

Results: The results are shown in Fig. 5. The reduction of the CD206-positive macrophages resulted in a decrease in the expression of genes (CXCL12, CXCL14, and Acta2) in the fibroblasts of the tumors, which genes suppress infiltration of CD8-positive T cells into a tumor. This change is thought to be a change that allows CD8-positive T cells to easily infiltrate a tumor.

The above results revealed that CD206-positive M2 macrophages contribute positively to the development/progression of a cancer, and that an anticancer effect can be obtained by reducing CD206-positive M2 macrophages. Thus, it was shown that a substance having an ability to remove CD206-positive M2 macrophages can be used as an effective component of a pharmaceutical composition for the prevention or treatment of a cancer. Further, it was shown that a substance having an ability to remove CD206-positive M2 macrophages can be used as an effective component of a composition for killing fibroblasts locating adjacent to a tumor tissue, a composition for promoting infiltration of cytotoxic T cells into a tumor tissue, a composition for suppressing the expression of a gene(s) in fibroblasts, which gene(s) suppress(es) infiltration of cytotoxic T cells into a tumor, a composition for promoting the growth of cytotoxic T cells, a composition for enhancing the cytotoxic activity of cytotoxic T cells, and a composition for enhancing the expression of a gene(s) associated with the cytotoxicity of cytotoxic T cells.

Examples of the substance having an ability to remove CD206-positive M2 macrophages include anti-CD206 antibodies. Examples of the anti-CD206 antibodies having an ability to remove CD206-positive M2 macrophages are shown below.

(Antigen)

Cultured cells (FreeStyle (registered trademark) 293-F cell) were allowed to express the extracellular domain (64 to 4161 in SEQ ID NO:118) of a mouse CD206 antigen, and its purified protein was used as the antigen.

(CD206-Deficient Mice)

B6.129P2-Mrc1<tm1Mnz>/J mice were purchased from Jackson Laboratory.

<Example 4: Preparation of Anti-CD206 Monoclonal Antibodies>

The CD206-deficient mice were immunized by administration of a mixture of the CD206 antigen and an adjuvant. Two weeks after the first antibody administration, the CD206 antigen was administered again. One week thereafter, the spleen was collected. After isolation of spleen cells, the cells were used for acquisition of CD206-specific antibodies using the ISAAC method, or stored at - 80°C.

By the ISAAC method, first screening of the spleen cells was carried out for lymphocytes producing antibodies that bind to CD206, to obtain 90 clones. Antibody cDNAs were cloned, and gene transfer into HEK293F cells was carried out to obtain antibody-producing cells. As a result, 13 kinds of mouse anti-human CD206 monoclonal antibodies were prepared.

The anti-CD206 antibodies prepared as described above, and an anti-CD206 antibody as a Comparative Example (the commercially available anti-CD206 antibody RATANTI MOUSE CD206 (manufactured by BIO-RAD)) were labeled using the Allophycocyanin Labeling Kit - NH2 (detection by APC)(manufactured by DOJINDO). On the other hand, gene transfer into cultured cells (HEK293T cells) was carried out to allow expression of CD206 and Venus (detection by FITC) (prepared by the Department of Molecular Neuroscience, University of Toyama), and the binding ability of each antibody to CD206-positive M2 macrophages was detected by flow cytometry. The results are shown in Fig. 6. Eleven kinds (Nos. 10, 27, 35, 40, 44, 47, 54, 59, 72, 73, and 83) with which the CD206-positive M2 macrophages could be clearly detected compared to the anti-CD206 antibody of Comparative Example were finally selected.

Further, as a result of ELISA using a plate on which a CD206 antigen (prepared by the Department of Molecular Neuroscience, University of Toyama) was immobilized, binding of the anti-CD206 antibodies of the present invention was found similarly to a case where an anti-CD206 control antibody (prepared by the Department of Immunology, University of Toyama) was used.

Thus, the anti-CD206 antibodies of the present invention were shown to have a CD206-binding ability.

The base sequences of the heavy-chain and light-chain variable regions of the 13 kinds of antibodies, and the base sequence of each CDR were analyzed. The amino acid sequences of the heavy-chain and light-chain variable regions of the antibodies obtained by these base sequences, and the amino acid sequence of each CDR are shown in Table 1 below and SEQUENCE LISTING.

Table 1 (Sequence and Sequence Number

| Antibody | | HCDR1 | HCDR2 | HCDR3 | LCDR1 | LCDR2 | LCDR3 | VH | VL |
|---|---|---|---|---|---|---|---|---|---|
| No. 10 | Amino acid Base | 27 | 28 | 29 | 30 | YTS | 31 | 1 | 2 |
| | | | | | | | | 92 | 93 |

(continued)

| Antibody | | HCDR1 | HCDR2 | HCDR3 | LCDR1 | LCDR2 | LCDR3 | VH | VL |
|---|---|---|---|---|---|---|---|---|---|
| No. 11 | Amino acid Base | 32 | 33 | 34 | 35 | KVS | 36 | 3 | 4 |
| | | | | | | | | 94 | 95 |
| No. 27 | Amino acid Base | 37 | 38 | 39 | 40 | LMS | 41 | 5 | 6 |
| | | | | | | | | 96 | 97 |
| No. 35 | Amino acid Base | 42 | 43 | 44 | 45 | RAS | 46 | 7 | 8 |
| | | | | | | | | 98 | 99 |
| No. 40 | Amino acid Base | 47 | 48 | 49 | 50 | FAS | 51 | 9 | 10 |
| | | | | | | | | 100 | 101 |
| No. 44 | Amino acid Base | 52 | 53 | 54 | 55 | AAT | 56 | 11 | 12 |
| | | | | | | | | 102 | 103 |
| No. 47 | Amino acid Base | 57 | 58 | 59 | 60 | YTS | 61 | 13 | 14 |
| | | | | | | | | 104 | 105 |
| No. 54 | Amino acid Base | 62 | 63 | 64 | 65 | KAS | 66 | 15 | 16 |
| | | | | | | | | 106 | 107 |
| No. 59 | Amino acid Base | 67 | 68 | 69 | 70 | YTS | 71 | 17 | 18 |
| | | | | | | | | 109 | 109 |
| No. 64 | Amino acid Base | 72 | 73 | 74 | 75 | SAS | 76 | 19 | 20 |
| | | | | | | | | 110 | 111 |
| No. 72 | Amino acid Base | 77 | 78 | 79 | 80 | ATS | 81 | 21 | 22 |
| | | | | | | | | 112 | 113 |
| No. 73 | Amino acid Base | 82 | 83 | 84 | 85 | RAN | 86 | 23 | 24 |
| | | | | | | | | 114 | 115 |
| No. 83 | Amino acid Base | 87 | 88 | 89 | 90 | YTS | 91 | 25 | 26 |
| | | | | | | | | 116 | 117 |

<Example 5: Evaluation of Complement-Dependent Cytotoxicities (CDCs) of Anti-CD206 Antibodies>

The CDC activities of the anti-CD206 antibodies prepared in Example 4 against CD206-positive cells were evaluated as follows.

More specifically, a CD206-positive fraction was collected by FACSAria (registered trademark) (manufactured by Becton, Dickinson and Company, Japan), and a CDC assay was carried out.

[CDC Assay]

1. The spleen was collected from one mouse.
2. Homogenization was carried out using RPMI1640.
3. A 70-μm cell strainer was placed in a 50-mL Falcon (registered trademark) tube, and isolated spleen cells were collected.
4. Centrifugation was performed at 4°C for 5 minutes at 1500 rpm.
5. The supernatant was discarded, and the cells were resuspended in 1 × Lysis Buffer.
6. Incubation was performed at RT for 10 minutes.
7. Centrifugation was performed at 4°C for 5 minutes at 1500 rpm, followed by discarding the supernatant.

8. After addition of 7.5 μL of CD3e-PE (for T cells), 5 μL of CD19-APCCY7 (for B cells), and 5 μL of NK1.1-FITC (for NK cells), staining was carried out on ice for 40 minutes.
9. After addition of 200 μL of FACS buffer, centrifugation was performed at 1500 rpm for 3 minutes (using a 96-well plate).
10. The supernatant was discarded (10. to 11. were repeated three times).
11. Stained cells such as T cells, and an unstained CD206-positive fraction were collected by FACSAria.
12. Centrifugation was performed at 4°C for 5 minutes at 1500 rpm, followed by discarding the supernatant.
13. The collected cells were plated in a 96-well plate.
14. An anti-CD206 antibody or an anti-CD206 control antibody (Hel2, Hel6, Hel 10 (prepared by the Department of Immunology, University of Toyama), rCD206 (manufactured by BIO-RAD), or MR (manufactured by Abcam)) was added.
15. A complement (Low Tox-M Rabbit complement (manufactured by Cedarlane Laboratories Ltd.)) was added (10% Comp).
16. Incubation was carried out at 37°C for 1 hr.
17. Staining was carried out using a secondary antibody (CD206-positive cells (APC) / dead cells (7AAD)).
18. The ratio between CD206-positive cells and CD206-positive 7AAD-positive cells was investigated by FACS.

The results are shown in Fig. 7. In the wells to which the anti-CD206 antibodies prepared in Example 4 were added, the ratio of live CD206-positive cells decreased, and the ratio of dead CD206-positive cells increased. Thus, the anti-CD206 antibodies prepared in Example 4 were shown to have a CDC activity against CD206-positive cells.

<Example 6: Evaluation of Antibody-Dependent Cell-Mediated Cytotoxicities (ADCCs) of Anti-CD206 Antibodies>

The ADCC activities of the anti-CD206 antibodies prepared in Example 4 against CD206-positive cells were evaluated as follows.
More specifically, a CD206-positive fraction was collected by FACSAria, and an ADCC assay was carried out.

[ADCC Assay]

1. The spleen was collected from one mouse.
2. Homogenization was carried out using RPMI1640.
3. A 70-μm cell strainer was placed in a 50-mL Falcon tube, and isolated spleen cells were collected.
4. Centrifugation was performed at 4°C for 5 minutes at 1500 rpm.
5. 4. The supernatant was discarded, and the cells were resuspended in 1 × Lysis Buffer.
6. Incubation was performed at RT for 10 minutes.
7. Centrifugation was performed at 4°C for 5 minutes at 1500 rpm, followed by discarding the supernatant.
8. After addition of 7.5 μL of CD3e-PE, 5 μL of CD19-APCCY7, and 5 μL of NK1.1-FITC, staining was carried out on ice for 40 minutes.
9. After addition of 200 μL of FACS buffer, centrifugation was performed at 1500 rpm for 3 minutes (using a 96-well plate).
10. The supernatant was discarded (10. to 11. were repeated three times).
11. Stained cells such as T cells, and an unstained CD206-positive fraction were collected by FACSAria.
12. Centrifugation was performed at 4°C for 5 minutes at 1500 rpm, followed by discarding the supernatant.
13. The collected cells were plated in a 96-well plate.
14. An anti-CD206 antibody or an anti-CD206 control antibody was added.
15. Incubation was carried out at 37°C for 12 hrs.
16. Staining was carried out using a secondary antibody (CD206-positive cells (APC) / dead cells (7AAD)).
17. The ratio between CD206-positive cells and CD206-positive 7AAD-positive cells was investigated by FACS.

The results are shown in Fig. 8. In the wells to which the anti-CD206 antibodies prepared in Example 4 were added, the ratio of live CD206-positive cells decreased, and the ratio of dead CD206-positive cells increased. Thus, the anti-CD206 antibodies prepared in Example 4 were shown to have a ADCC activity against CD206-positive cells.

<Example 7: Evaluation of Cytotoxic Actions of Anti-CD206 Antibodies against CD206-Positive M2 Macrophages in Biological Tissues>

The cytotoxic actions of anti-CD206 antibodies prepared in Example 4 against CD206-positive M2 macrophages in biological tissues were evaluated as follows.

A dilution of each test antibody in PBS, or PBS as a control, was intraperitoneally administered to C57BL/6J mice (manufactured by CLEA Japan, Inc.) twice at a 48-hour interval at a concentration of 1 μg/mouse body weight (g) (No.47, n=4; No. 83, n=5). Four days (96 hours) after the administration, the spleen, visceral fat, and subcutaneous fat were collected from the mice, and RNA was extracted therefrom, followed by performing qPCR for the F4/80, CD11c, and CD206 genes, as described below. Further, cells of the spleen, visceral fat (epididymal white adipose tissue: eWAT), and inguinal subcutaneous fat (inguinal white adipose tissue: iWAT) were collected from the mice, and subjected to FACS analysis.

As a result, according to the qPCR results shown in Fig. 9, trends toward a decrease in CD206-positive M2 macrophages (indicated by arrows) could be found in the visceral fat in the groups in which the anti-CD206 antibodies were administered. Further, according to the results of the FACS analysis shown in Fig. 10, significant decreases in CD206-positive M2 macrophages could be found in the visceral fat and the subcutaneous fat in the groups in which the anti-CD206 antibodies were administered. Thus, the anti-CD206 antibodies prepared in Example 4 were shown to have a cytotoxic action against CD206-positive M2 macrophages especially in biological tissues where CD206-positive M2 macrophages accumulate.

[qPCR]

1. The spleen, visceral fat, and subcutaneous fat were collected from mice.
2. The tissues were homogenized, and then RNA was extracted using the Qiagen RNA extraction kit.
3. The RNA concentration was measured.
4. cDNA was prepared using the TaKaRa Prime Script (registered trademark) ET reagent kit.

(Reverse Transcription Reaction)

37°C, 15 minutes (reverse transcription reaction)
85°C, 5 seconds (heat inactivation of reverse transcriptase)
4°C, ∞

5. Real Time PCR (SYBR)

(1) Ice and samples (cDNAs) were provided.
(2) A control and primers for the investigation were taken out from a -20°C freezer, and placed on aluminum foil.
(3) A master mixture was taken out from a -20°C freezer, and placed on ice.
(4) Micro Amp tubes and lids were provided.
(5) Pale-yellow tubes in the same number as the primers were provided, and the primer names were written on the lids.
(6) Mixtures of the master mixture and the primers were provided in the pale-yellow tubes.

Master mixture: 12.5 μL (7.5) × sample number = μL
$H_2O$, PCR Grade: 9 μL (5.4) × sample number = μL
Primer FW: 0.5 μL (0.4) × sample number = μL
Primer RV: 0.5 μL (0.4) × sample number = μL
ROX Reference Dye II: 0.5 μL (0.3) × sample number = μL
*The primers were added with pipetting to the tubes in which the master mixture was placed.
After thorough mixing, the resulting mixture was spun down.

(7) The prepared mixture was aliquoted in 23-μL volumes into the Micro Amp tubes.
(8) After the aliquoting of the mixture in 23-μL volumes, 2 μL of each sample was added to each aliquot to achieve a total volume of 25 μL.

$$(7)\ 23\ \mu L + (8)\ 2\ \mu L = 25\ \mu L$$

(9) The Micro Amps were closed with the lids. After confirming the absence of air bubbles, and tight sealing by the lids, the liquid remaining on the side wall was spun down.
(10) The tubes were placed in a real-time quantitative PCR system (MX3000P (manufactured by Agilent Technologies, Inc.)).

6. The results from MX3000P were analyzed.

(Krebs-Hense-leit-HEPES (KRHAG) buffer)
Physiological saline, 5% BSA in physiological saline, 1 M KCl, 1 M $CaCl_2$, 1 M $KH_2PO4$, 1 M $MgSO_4$, 200 mM Hepes, 200 mM glucose
(Collagenase)
18 mg in 9 mL KRHAG (taken in advance and stored at -30°C; added immediately before the use)

[FACS Analysis]

(Collection of Adipose Tissue Stromal Vascular Cells (Stromal Vascular Fraction (SVF))

1. White adipose tissue (WAT) was collected.
2. Mincing was carried out.
3. The sample was placed in the collagenase (37°C, 27 times/min).
4. Tapping was carried out at minute 20 and minute 40.
5. At minute 45, on ice
6. After adding 4 mL of KRHAG, filtration was carried out through stocking.
7. 1500 rpm 5 min 4°C
8. The supernatant was sucked. The pellet was transferred to a 2-mL tube, leaving 2 mL of the pellet.
9. 1500 rpm 5 min 4°C
10. The supernatant was sucked, and 2 mL of KRHAG was added.
11. 1500 rpm 5 min 4°C
12. The supernatant was sucked, and 2 mL of KRHAG was added.
13. 1500 rpm 5 min 4°C
14. The supernatant was sucked, and 150 μL of FACS buffer was added.
15. lyse Lysis Buffer 1.35 mL
16. protect from light 15 min 4°C
17. 1500 rpm 5 min 4°C
18. The supernatant was discarded; 1 mL of FACS buffer.

(Collection of Spleen Cells)

1. The spleen was collected.
2. Homogenization was carried out.
3. 1500 rpm 5 min 4°C
4. The supernatant was sucked, and 150 μL of FACS buffer was added.
5. lyse Lysis Buffer 1.35 mL
6. protect from light 15 min 4°C
7. 1500 rpm 5 min 4°C
8. The supernatant was discarded; 1 mL of FACS buffer.

(Staining)

1. FC Brock : 2% FCS / PBS = (1:100) was added followed by 15 min 4°C.
2. CD45-PECy7 : 2% FCS/PBS = (1:400)

   F4/80-APCCy7 : 2% FCS/PBS = (1:80)
   CD11c-PE: 2% FCS/PBS = (1:50)
   CD206-APC : 2% FCS/PBS = (1:80) was added followed by 30 min 4°C.

3. Addition of 200 μL of 2% FCS/PBS
1500 rpm 5 min 4°C
4. The supernatant was discarded followed by addition of 200 μL of 2% FCS/PBS.
5. 1500 rpm 5 min 4°C
6. The supernatant was discarded followed by addition of 200 μL of 2% FCS/PBS.
7. Measurement was carried out by FACS (1 μL of 7AAD was added immediately before the measurement).

INDUSTRIAL APPLICABILITY

The present invention can be used as a pharmaceutical composition for the prevention or treatment of a cancer.

<Description of Sequence Listing>

SEQ ID NO:1 The amino acid sequence of the heavy-chain variable region of the anti-CD206 antibody (No. 10)
SEQ ID NO:2 The amino acid sequence of the light-chain variable region of the anti-CD206 antibody (No. 10)
SEQ ID NO:3 The amino acid sequence of the heavy-chain variable region of the anti-CD206 antibody (No. 11)
SEQ ID NO:4 The amino acid sequence of the light-chain variable region of the anti-CD206 antibody (No. 11)
SEQ ID NO:5 The amino acid sequence of the heavy-chain variable region of the anti-CD206 antibody (No. 27)
SEQ ID NO:6 The amino acid sequence of the light-chain variable region of the anti-CD206 antibody (No. 27)
SEQ ID NO:7 The amino acid sequence of the heavy-chain variable region of the anti-CD206 antibody (No. 35)
SEQ ID NO:8 The amino acid sequence of the light-chain variable region of the anti-CD206 antibody (No. 35)
SEQ ID NO:9 The amino acid sequence of the heavy-chain variable region of the anti-CD206 antibody (No. 40)
SEQ ID NO:10 The amino acid sequence of the light-chain variable region of the anti-CD206 antibody (No. 40)
SEQ ID NO:11 The amino acid sequence of the heavy-chain variable region of the anti-CD206 antibody (No. 44)
SEQ ID NO:12 The amino acid sequence of the light-chain variable region of the anti-CD206 antibody (No. 44)
SEQ ID NO:13 The amino acid sequence of the heavy-chain variable region of the anti-CD206 antibody (No. 47)
SEQ ID NO:14 The amino acid sequence of the light-chain variable region of the anti-CD206 antibody (No. 47)
SEQ ID NO:15 The amino acid sequence of the heavy-chain variable region of the anti-CD206 antibody (No. 54)
SEQ ID NO:16 The amino acid sequence of the light-chain variable region of the anti-CD206 antibody (No. 54)
SEQ ID NO:17 The amino acid sequence of the heavy-chain variable region of the anti-CD206 antibody (No. 59)
SEQ ID NO:18 The amino acid sequence of the light-chain variable region of the anti-CD206 antibody (No. 59)
SEQ ID NO:19 The amino acid sequence of the heavy-chain variable region of the anti-CD206 antibody (No. 64)
SEQ ID NO:20 The amino acid sequence of the light-chain variable region of the anti-CD206 antibody (No. 64)
SEQ ID NO:21 The amino acid sequence of the heavy-chain variable region of the anti-CD206 antibody (No. 72)
SEQ ID NO:22 The amino acid sequence of the light-chain variable region of the anti-CD206 antibody (No. 72)
SEQ ID NO:23 The amino acid sequence of the heavy-chain variable region of the anti-CD206 antibody (No. 73)
SEQ ID NO:24 The amino acid sequence of the light-chain variable region of the anti-CD206 antibody (No. 73)
SEQ ID NO:25 The amino acid sequence of the heavy-chain variable region of the anti-CD206 antibody (No. 83)
SEQ ID NO:26 The amino acid sequence of the light-chain variable region of the anti-CD206 antibody (No. 83)
SEQ ID NO:27 The amino acid sequence of HCDR1 of the anti-CD206 antibody (No.10)
SEQ ID NO:28 The amino acid sequence of HCDR2 of the anti-CD206 antibody (No. 10)
SEQ ID NO:29 The amino acid sequence of HCDR3 of the anti-CD206 antibody (No. 10)
SEQ ID NO:30 The amino acid sequence of LCDR1 of the anti-CD206 antibody (No. 10)
SEQ ID NO:31 The amino acid sequence of LCDR3 of the anti-CD206 antibody (No. 10)
SEQ ID NO:32 The amino acid sequence of HCDR1 of the anti-CD206 antibody (No. 11)
SEQ ID NO:33 The amino acid sequence of HCDR2 of the anti-CD206 antibody (No. 11)
SEQ ID NO:34 The amino acid sequence of HCDR3 of the anti-CD206 antibody (No. 11)
SEQ ID NO:35 The amino acid sequence of LCDR1 of the anti-CD206 antibody (No. 11)
SEQ ID NO:36 The amino acid sequence of LCDR3 of the anti-CD206 antibody (No. 11)

SEQ ID NO:37 The amino acid sequence of HCDR1 of the anti-CD206 antibody (No. 27)
SEQ ID NO:38 The amino acid sequence of HCDR2 of the anti-CD206 antibody (No. 27)
SEQ ID NO:39 The amino acid sequence of HCDR3 of the anti-CD206 antibody (No. 27)
SEQ ID NO:40 The amino acid sequence of LCDR1 of the anti-CD206 antibody (No. 27)
SEQ ID NO:41 The amino acid sequence of LCDR3 of the anti-CD206 antibody (No. 27)
SEQ ID NO:42 The amino acid sequence of HCDR1 of the anti-CD206 antibody (No. 35)
SEQ ID NO:43 The amino acid sequence of HCDR2 of the anti-CD206 antibody (No. 35)
SEQ ID NO:44 The amino acid sequence of HCDR3 of the anti-CD206 antibody (No. 35)
SEQ ID NO:45 The amino acid sequence of LCDR1 of the anti-CD206 antibody (No. 35)
SEQ ID NO:46 The amino acid sequence of LCDR3 of the anti-CD206 antibody (No. 35)
SEQ ID NO:47 The amino acid sequence of HCDR1 of the anti-CD206 antibody (No. 40)
SEQ ID NO:48 The amino acid sequence of HCDR2 of the anti-CD206 antibody (No. 40)
SEQ ID NO:49 The amino acid sequence of HCDR3 of the anti-CD206 antibody (No. 40)
SEQ ID NO:50 The amino acid sequence of LCDR1 of the anti-CD206 antibody (No. 40)
SEQ ID NO:51 The amino acid sequence of LCDR3 of the anti-CD206 antibody (No. 40)
SEQ ID NO:52 The amino acid sequence of HCDR1 of the anti-CD206 antibody (No. 44)
SEQ ID NO:53 The amino acid sequence of HCDR2 of the anti-CD206 antibody (No. 44)
SEQ ID NO:54 The amino acid sequence of HCDR3 of the anti-CD206 antibody (No. 44)
SEQ ID NO:55 The amino acid sequence of LCDR1 of the anti-CD206 antibody (No. 44)
SEQ ID NO:56 The amino acid sequence of LCDR3 of the anti-CD206 antibody (No. 44)
SEQ ID NO:57 The amino acid sequence of HCDR1 of the anti-CD206 antibody (No. 47)
SEQ ID NO:58 The amino acid sequence of HCDR2 of the anti-CD206 antibody (No. 47)
SEQ ID NO:59 The amino acid sequence of HCDR3 of the anti-CD206 antibody (No. 47)
SEQ ID NO:60 The amino acid sequence of LCDR1 of the anti-CD206 antibody (No. 47)
SEQ ID NO:61 The amino acid sequence of LCDR3 of the anti-CD206 antibody (No. 47)
SEQ ID NO:62 The amino acid sequence of HCDR1 of the anti-CD206 antibody (No. 54)
SEQ ID NO:63 The amino acid sequence of HCDR2 of the anti-CD206 antibody (No. 54)
SEQ ID NO:64 The amino acid sequence of HCDR3 of the anti-CD206 antibody (No. 54)
SEQ ID NO:65 The amino acid sequence of LCDR1 of the anti-CD206 antibody (No. 54)
SEQ ID NO:66 The amino acid sequence of LCDR3 of the anti-CD206 antibody (No. 54)
SEQ ID NO:67 The amino acid sequence of HCDR1 of the anti-CD206 antibody (No. 59)
SEQ ID NO:68 The amino acid sequence of HCDR2 of the anti-CD206 antibody (No. 59)
SEQ ID NO:69 The amino acid sequence of HCDR3 of the anti-CD206 antibody (No. 59)
SEQ ID NO:70 The amino acid sequence of LCDR1 of the anti-CD206 antibody (No. 59)
SEQ ID NO:71 The amino acid sequence of LCDR3 of the anti-CD206 antibody (No. 59)
SEQ ID NO:72 The amino acid sequence of HCDR1 of the anti-CD206 antibody (No. 64)
SEQ ID NO:73 The amino acid sequence of HCDR2 of the anti-CD206 antibody (No. 64)
SEQ ID NO:74 The amino acid sequence of HCDR3 of the anti-CD206 antibody (No. 64)
SEQ ID NO:75 The amino acid sequence of LCDR1 of the anti-CD206 antibody (No. 64)
SEQ ID NO:76 The amino acid sequence of LCDR3 of the anti-CD206 antibody (No. 64)
SEQ ID NO:77 The amino acid sequence of HCDR1 of the anti-CD206 antibody (No. 72)
SEQ ID NO:78 The amino acid sequence of HCDR2 of the anti-CD206 antibody (No. 72)
SEQ ID NO:79 The amino acid sequence of HCDR3 of the anti-CD206 antibody (No. 72)
SEQ ID NO:80 The amino acid sequence of LCDR1 of the anti-CD206 antibody (No. 72)
SEQ ID NO:81 The amino acid sequence of LCDR3 of the anti-CD206 antibody (No. 72)
SEQ ID NO:82 The amino acid sequence of HCDR1 of the anti-CD206 antibody (No. 73)
SEQ ID NO:83 The amino acid sequence of HCDR2 of the anti-CD206 antibody (No. 73)
SEQ ID NO:84 The amino acid sequence of HCDR3 of the anti-CD206 antibody (No. 73)
SEQ ID NO:85 The amino acid sequence of LCDR1 of the anti-CD206 antibody (No. 73)
SEQ ID NO:86 The amino acid sequence of LCDR3 of the anti-CD206 antibody (No. 73)
SEQ ID NO:87 The amino acid sequence of HCDR1 of the anti-CD206 antibody (No. 83)
SEQ ID NO:88 The amino acid sequence of HCDR2 of the anti-CD206 antibody (No. 83)
SEQ ID NO:89 The amino acid sequence of HCDR3 of the anti-CD206 antibody (No. 83)
SEQ ID NO:90 The amino acid sequence of LCDR1 of the anti-CD206 antibody (No. 83)
SEQ ID NO:91 The amino acid sequence of LCDR3 of the anti-CD206 antibody (No. 83)
SEQ ID NO:92 The base sequence encoding the heavy-chain variable region of the anti-CD206 antibody (No. 10)
SEQ ID NO:93 The base sequence encoding the light-chain variable region of the anti-CD206 antibody (No. 10)
SEQ ID NO:94 The base sequence encoding the heavy-chain variable region of the anti-CD206 antibody (No. 11)

SEQ ID NO:95 The base sequence encoding the light-chain variable region of the anti-CD206 antibody (No. 11)
SEQ ID NO:96 The base sequence encoding the heavy-chain variable region of the anti-CD206 antibody (No. 27)
SEQ ID NO:97 The base sequence encoding the light-chain variable region of the anti-CD206 antibody (No. 27)
SEQ ID NO:98 The base sequence encoding the heavy-chain variable region of the anti-CD206 antibody (No. 35)
SEQ ID NO:99 The base sequence encoding the light-chain variable region of the anti-CD206 antibody (No. 35)
SEQ ID NO:100 The base sequence encoding the heavy-chain variable region of the anti-CD206 antibody (No. 40)
SEQ ID NO:101 The base sequence encoding the light-chain variable region of the anti-CD206 antibody (No. 40)
SEQ ID NO:102 The base sequence encoding the heavy-chain variable region of the anti-CD206 antibody (No. 44)
SEQ ID NO:103 The base sequence encoding the light-chain variable region of the anti-CD206 antibody (No. 44)
SEQ ID NO:104 The base sequence encoding the heavy-chain variable region of the anti-CD206 antibody (No. 47)
SEQ ID NO:105 The base sequence encoding the light-chain variable region of the anti-CD206 antibody (No. 47)
SEQ ID NO:106 The base sequence encoding the heavy-chain variable region of the anti-CD206 antibody (No. 54)
SEQ ID NO:107 The base sequence encoding the light-chain variable region of the anti-CD206 antibody (No. 54)
SEQ ID NO:108 The base sequence encoding the heavy-chain variable region of the anti-CD206 antibody (No. 59)
SEQ ID NO:109 The base sequence encoding the light-chain variable region of the anti-CD206 antibody (No. 59)
SEQ ID NO:110 The base sequence encoding the heavy-chain variable region of the anti-CD206 antibody (No. 64)
SEQ ID NO:111 The base sequence encoding the light-chain variable region of the anti-CD206 antibody (No. 64)
SEQ ID NO:112 The base sequence encoding the heavy-chain variable region of the anti-CD206 antibody (No. 72)
SEQ ID NO:113 The base sequence encoding the light-chain variable region of the anti-CD206 antibody (No. 72)
SEQ ID NO:114 The base sequence encoding the heavy-chain variable region of the anti-CD206 antibody (No. 73)
SEQ ID NO:115 The base sequence encoding the light-chain variable region of the anti-CD206 antibody (No. 73)
SEQ ID NO:116 The base sequence encoding the heavy-chain variable region of the anti-CD206 antibody (No. 83)
SEQ ID NO:117 The base sequence encoding the light-chain variable region of the anti-CD206 antibody (No. 83)
SEQ ID NO:118 The base sequence of the mouse CD206 coding region

## Claims

1. A composition for killing fibroblasts locating adjacent to a tumor tissue, wherein the composition comprising, as an effective component, a substance having an ability to kill CD206-positive M2 macrophages.

2. A composition for promoting infiltration of cytotoxic T cells into a tumor tissue, wherein the composition comprising, as an effective component, a substance having an ability to kill CD206-positive M2 macrophages.

3. The composition for promoting infiltration of cytotoxic T cells into a tumor tissue according to claim 2, wherein the promotion of infiltration of the cytotoxic T cells into the tumor tissue involves reduction of fibroblasts locating adjacent to the tumor tissue.

4. A composition for suppressing expression of one or more genes selected from Acta2, CXCL12, and CXCL14 on fibroblasts locating adjacent to a tumor tissue, wherein the composition comprising, as an effective component, a substance having an ability to kill CD206-positive M2 macrophages.

5. A composition for promoting growth of cytotoxic T cells, wherein the composition comprising, as an effective component, a substance having an ability to kill CD206-positive M2 macrophages.

6. A composition for enhancing cytotoxic activity of cytotoxic T cells, wherein the composition comprising, as an effective component, a substance having an ability to kill CD206-positive M2 macrophages.

7. A composition for enhancing expression of one or more genes selected from TNF$\alpha$, IFN$\gamma$, and Gzmb, wherein the composition comprising, as an effective component, a substance having an ability to kill CD206-positive M2 macrophages.

8. A pharmaceutical composition for prevention or treatment of a cancer, wherein the pharmaceutical composition comprising, as an effective component, a substance having an ability to kill CD206-positive M2 macrophages.

9. The pharmaceutical composition for the prevention or treatment of a cancer according to claim 8, wherein the substance having an ability to kill CD206-positive M2 macrophages is an anti-CD206 antibody or a functional fragment thereof.

**10.** The pharmaceutical composition for the prevention or treatment of a cancer according to claim 9, wherein the antibody is an antibody selected from the following:

(a) an antibody containing:

heavy-chain HCDR1 having the amino acid sequence of SEQ ID NO:27;
heavy-chain HCDR2 having the amino acid sequence of SEQ ID NO:28;
heavy-chain HCDR3 having the amino acid sequence of SEQ ID NO:29;
light-chain LCDR1 having the amino acid sequence of SEQ ID NO:30;
light-chain LCDR2 having the amino acid sequence of YTS; and
light-chain LCDR3 having the amino acid sequence of SEQ ID NO:31;

(b) an antibody containing:

heavy-chain HCDR1 having the amino acid sequence of SEQ ID NO:37;
heavy-chain HCDR2 having the amino acid sequence of SEQ ID NO:38;
heavy-chain HCDR3 having the amino acid sequence of SEQ ID NO:39;
light-chain LCDR1 having the amino acid sequence of SEQ ID NO:40;
light-chain LCDR2 having the amino acid sequence of LMS; and
light-chain LCDR3 having the amino acid sequence of SEQ ID NO:41;

(c) an antibody containing:

heavy-chain HCDR1 having the amino acid sequence of SEQ ID NO:42;
heavy-chain HCDR2 having the amino acid sequence of SEQ ID NO:43;
heavy-chain HCDR3 having the amino acid sequence of SEQ ID NO:44;
light-chain LCDR1 having the amino acid sequence of SEQ ID NO:45;
light-chain LCDR2 having the amino acid sequence of RAS; and
light-chain LCDR3 having the amino acid sequence of SEQ ID NO:46;

(d) an antibody containing:

heavy-chain HCDR1 having the amino acid sequence of SEQ ID NO:47;
heavy-chain HCDR2 having the amino acid sequence of SEQ ID NO:48;
heavy-chain HCDR3 having the amino acid sequence of SEQ ID NO:49;
light-chain LCDR1 having the amino acid sequence of SEQ ID NO:50;
light-chain LCDR2 having the amino acid sequence of FAS; and
light-chain LCDR3 having the amino acid sequence of SEQ ID NO:51;

(e) an antibody containing:

heavy-chain HCDR1 having the amino acid sequence of SEQ ID NO:52;
heavy-chain HCDR2 having the amino acid sequence of SEQ ID NO:53;
heavy-chain HCDR3 having the amino acid sequence of SEQ ID NO:54;
light-chain LCDR1 having the amino acid sequence of SEQ ID NO:55;
light-chain LCDR2 having the amino acid sequence of AAT; and
light-chain LCDR3 having the amino acid sequence of SEQ ID NO:56;

(f) an antibody containing:

heavy-chain HCDR1 having the amino acid sequence of SEQ ID NO:57;
heavy-chain HCDR2 having the amino acid sequence of SEQ ID NO:58;
heavy-chain HCDR3 having the amino acid sequence of SEQ ID NO:59;
light-chain LCDR1 having the amino acid sequence of SEQ ID NO:60;
light-chain LCDR2 having the amino acid sequence of YTS; and
light-chain LCDR3 having the amino acid sequence of SEQ ID NO:61;

(g) an antibody containing:

heavy-chain HCDR1 having the amino acid sequence of SEQ ID NO:62;
heavy-chain HCDR2 having the amino acid sequence of SEQ ID NO:63;
heavy-chain HCDR3 having the amino acid sequence of SEQ ID NO:64;
light-chain LCDR1 having the amino acid sequence of SEQ ID NO:65;
light-chain LCDR2 having the amino acid sequence of KAS; and
light-chain LCDR3 having the amino acid sequence of SEQ ID NO:66;

(h) an antibody containing:

heavy-chain HCDR1 having the amino acid sequence of SEQ ID NO:67;
heavy-chain HCDR2 having the amino acid sequence of SEQ ID NO:68;
heavy-chain HCDR3 having the amino acid sequence of SEQ ID NO:69;
light-chain LCDR1 having the amino acid sequence of SEQ ID NO:70;
light-chain LCDR2 having the amino acid sequence of YTS; and
light-chain LCDR3 having the amino acid sequence of SEQ ID NO:71;

(i) an antibody containing:

heavy-chain HCDR1 having the amino acid sequence of SEQ ID NO:77;
heavy-chain HCDR2 having the amino acid sequence of SEQ ID NO:78;
heavy-chain HCDR3 having the amino acid sequence of SEQ ID NO:79;
light-chain LCDR1 having the amino acid sequence of SEQ ID NO:80;
light-chain LCDR2 having the amino acid sequence of ATS; and
light-chain LCDR3 having the amino acid sequence of SEQ ID NO:81;

(j) an antibody containing:

heavy-chain HCDR1 having the amino acid sequence of SEQ ID NO:82;
heavy-chain HCDR2 having the amino acid sequence of SEQ ID NO:83;
heavy-chain HCDR3 having the amino acid sequence of SEQ ID NO:84;
light-chain LCDR1 having the amino acid sequence of SEQ ID NO:85;
light-chain LCDR2 having the amino acid sequence of RAN; and
light-chain LCDR3 having the amino acid sequence of SEQ ID NO:86; and

(k) an antibody containing:

heavy-chain HCDR1 having the amino acid sequence of SEQ ID NO:87;
heavy-chain HCDR2 having the amino acid sequence of SEQ ID NO:88;
heavy-chain HCDR3 having the amino acid sequence of SEQ ID NO:89;
light-chain LCDR1 having the amino acid sequence of SEQ ID NO:90;
light-chain LCDR2 having the amino acid sequence of YTS; and
light-chain LCDR3 having the amino acid sequence of SEQ ID NO:91.

**11.** The pharmaceutical composition for the prevention or treatment of a cancer according to claim 9 or 10, wherein the antibody is an antibody selected from the following:

(A) an antibody containing:

a heavy-chain variable region having the amino acid sequence of SEQ ID NO:1, or a heavy-chain variable region having an amino acid sequence with a sequence identity of not less than 90% to the amino acid sequence and having a CD206-binding activity; and
a light-chain variable region having the amino acid sequence of SEQ ID NO:2, or a light-chain variable region having an amino acid sequence with a sequence identity of not less than 90% to the amino acid sequence and having a CD206-binding activity;

(B) an antibody containing:

a heavy-chain variable region having the amino acid sequence of SEQ ID NO:5, or a heavy-chain variable region having an amino acid sequence with a sequence identity of not less than 90% to the amino acid sequence and having a CD206-binding activity; and
a light-chain variable region having the amino acid sequence of SEQ ID NO:6, or a light-chain variable region having an amino acid sequence with a sequence identity of not less than 90% to the amino acid sequence and having a CD206-binding activity;

(C) an antibody containing:

a heavy-chain variable region having the amino acid sequence of SEQ ID NO:7, or a heavy-chain variable region having an amino acid sequence with a sequence identity of not less than 90% to the amino acid sequence and having a CD206-binding activity; and
a light-chain variable region having the amino acid sequence of SEQ ID NO:8, or a light-chain variable region having an amino acid sequence with a sequence identity of not less than 90% to the amino acid sequence and having a CD206-binding activity;

(D) an antibody containing:

a heavy-chain variable region having the amino acid sequence of SEQ ID NO:9, or a heavy-chain variable region having an amino acid sequence with a sequence identity of not less than 90% to the amino acid sequence and having a CD206-binding activity; and
a light-chain variable region having the amino acid sequence of SEQ ID NO:10, or a light-chain variable region having an amino acid sequence with a sequence identity of not less than 90% to the amino acid sequence and having a CD206-binding activity;

(E) an antibody containing:

a heavy-chain variable region having the amino acid sequence of SEQ ID NO:11, or a heavy-chain variable region having an amino acid sequence with a sequence identity of not less than 90% to the amino acid sequence and having a CD206-binding activity; and
a light-chain variable region having the amino acid sequence of SEQ ID NO:12, or a light-chain variable region having an amino acid sequence with a sequence identity of not less than 90% to the amino acid sequence and having a CD206-binding activity;

(F) an antibody containing:

a heavy-chain variable region having the amino acid sequence of SEQ ID NO:13, or a heavy-chain variable region having an amino acid sequence with a sequence identity of not less than 90% to the amino acid sequence and having a CD206-binding activity; and
a light-chain variable region having the amino acid sequence of SEQ ID NO:14, or a light-chain variable region having an amino acid sequence with a sequence identity of not less than 90% to the amino acid sequence and having a CD206-binding activity;

(G) an antibody containing:

a heavy-chain variable region having the amino acid sequence of SEQ ID NO:15, or a heavy-chain variable region having an amino acid sequence with a sequence identity of not less than 90% to the amino acid sequence and having a CD206-binding activity; and
a light-chain variable region having the amino acid sequence of SEQ ID NO:16, or a light-chain variable region having an amino acid sequence with a sequence identity of not less than 90% to the amino acid sequence and having a CD206-binding activity;

(H) an antibody containing:

a heavy-chain variable region having the amino acid sequence of SEQ ID NO:17, or a heavy-chain variable region having an amino acid sequence with a sequence identity of not less than 90% to the amino acid sequence and having a CD206-binding activity; and
a light-chain variable region having the amino acid sequence of SEQ ID NO:18, or a light-chain variable region having an amino acid sequence with a sequence identity of not less than 90% to the amino acid sequence and having a CD206-binding activity;

(I) an antibody containing:

a heavy-chain variable region having the amino acid sequence of SEQ ID NO:21, or a heavy-chain variable region having an amino acid sequence with a sequence identity of not less than 90% to the amino acid sequence and having a CD206-binding activity; and
a light-chain variable region having the amino acid sequence of SEQ ID NO:22, or a light-chain variable region having an amino acid sequence with a sequence identity of not less than 90% to the amino acid sequence and having a CD206-binding activity;

(J) an antibody containing:

a heavy-chain variable region having the amino acid sequence of SEQ ID NO:25, or a heavy-chain variable region having an amino acid sequence with a sequence identity of not less than 90% to the amino acid sequence and having a CD206-binding activity; and
a light-chain variable region having the amino acid sequence of SEQ ID NO:26, or a light-chain variable region having an amino acid sequence with a sequence identity of not less than 90% to the amino acid sequence and having a CD206-binding activity; and

(K) an antibody containing:

a heavy-chain variable region having the amino acid sequence of SEQ ID NO:25, or a heavy-chain variable region having an amino acid sequence with a sequence identity of not less than 90% to the amino acid sequence and having a CD206-binding activity; and
a light-chain variable region having the amino acid sequence of SEQ ID NO:26, or a light-chain variable region having an amino acid sequence with a sequence identity of not less than 90% to the amino acid sequence and having a CD206-binding activity.

Fig. 1

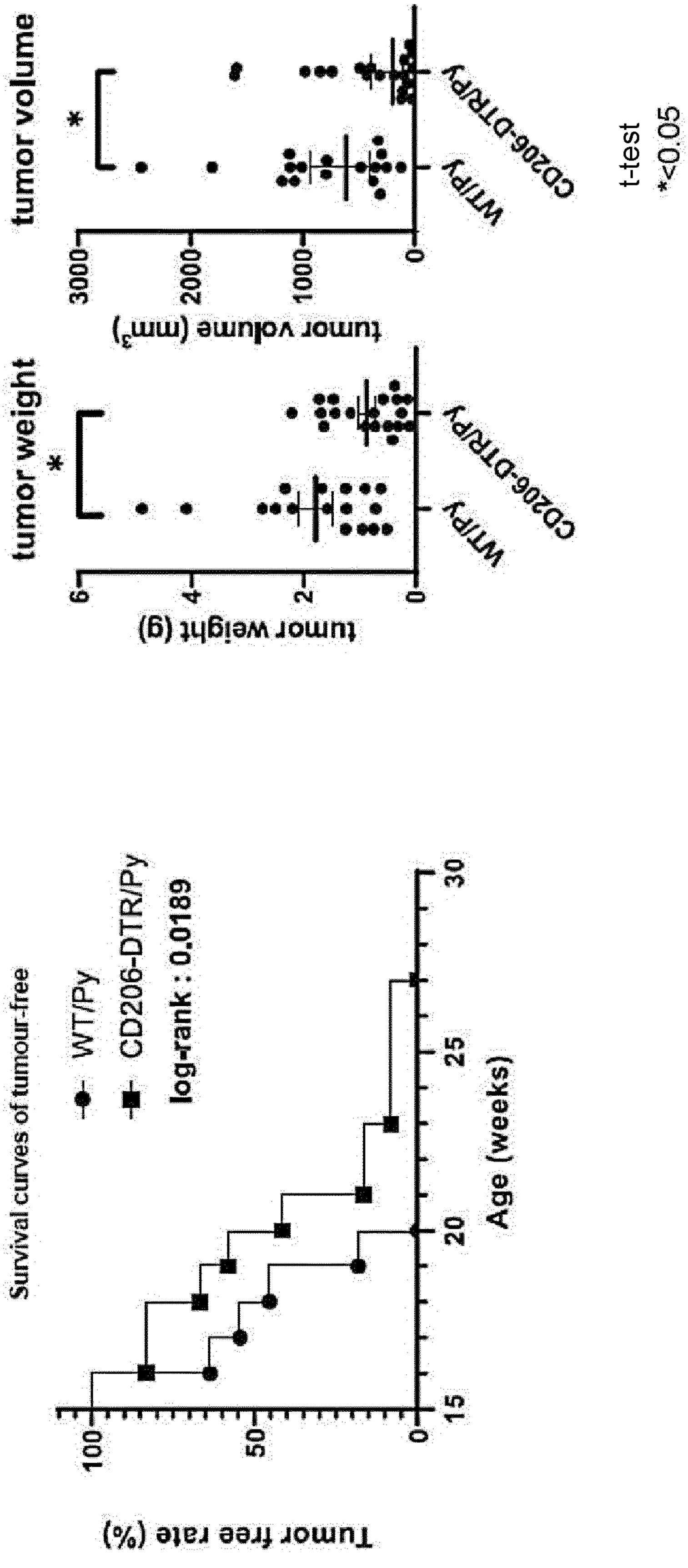

Survival curves of tumour-free
WT/Py
CD206-DTR/Py
log-rank : 0.0189
Tumor free rate (%)
100
50
0
15
20
25
30
Age (weeks)
tumor weight
tumor weight (g)
6
4
2
0
WT/Py
CD206-DTR/Py
*
tumor volume
tumor volume (mm$^3$)
3000
2000
1000
0
WT/Py
CD206-DTR/Py
*
t-test
*<0.05

Fig. 2

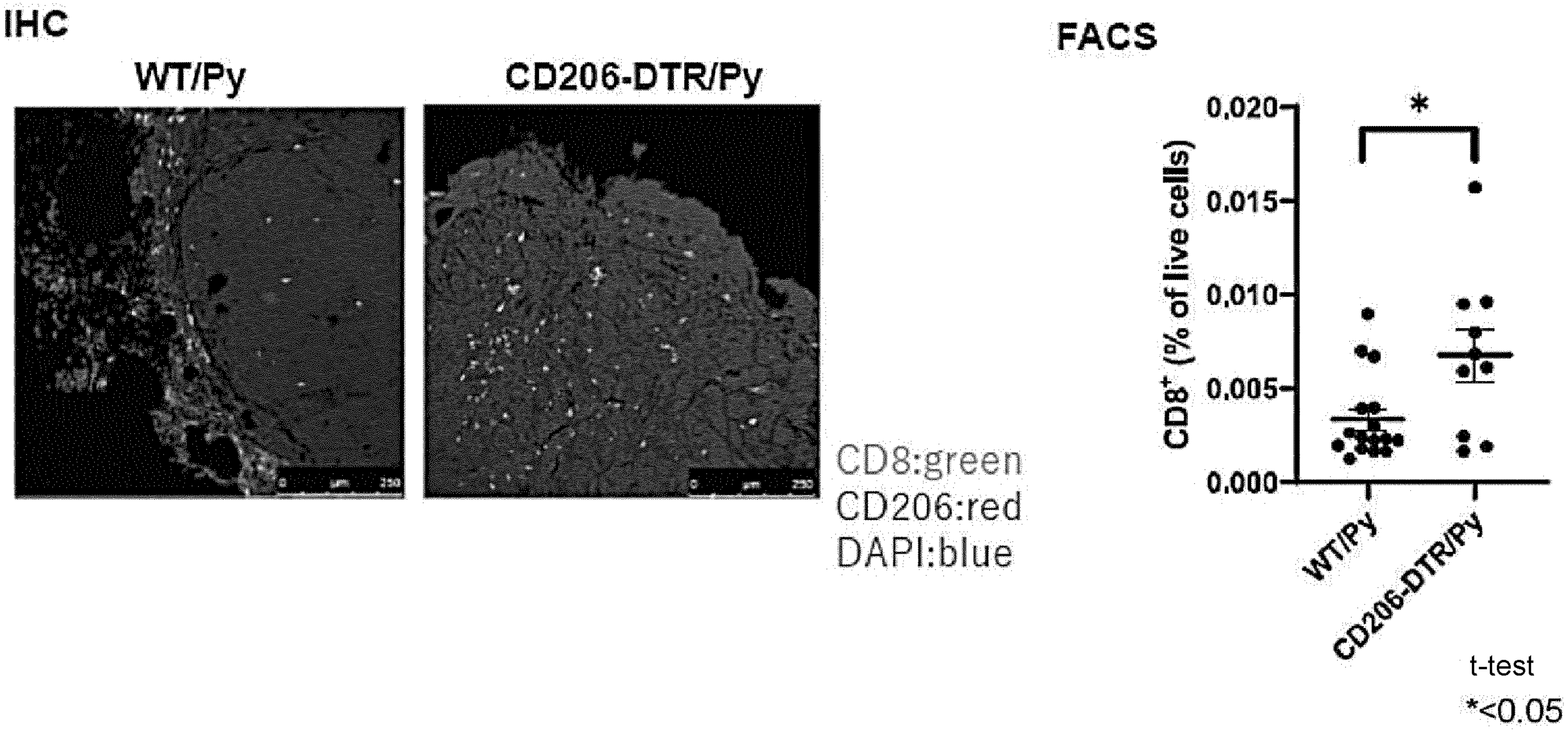
IHC
WT/Py
CD206-DTR/Py
CD8:green
CD206:red
DAPI:blue
FACS
CD8+ (% of live cells)
0.020
0.015
0.010
0.005
0.000
*
WT/Py
CD206-DTR/Py
t-test
*<0.05

Fig. 3

Quantitative RT-PCR analysis

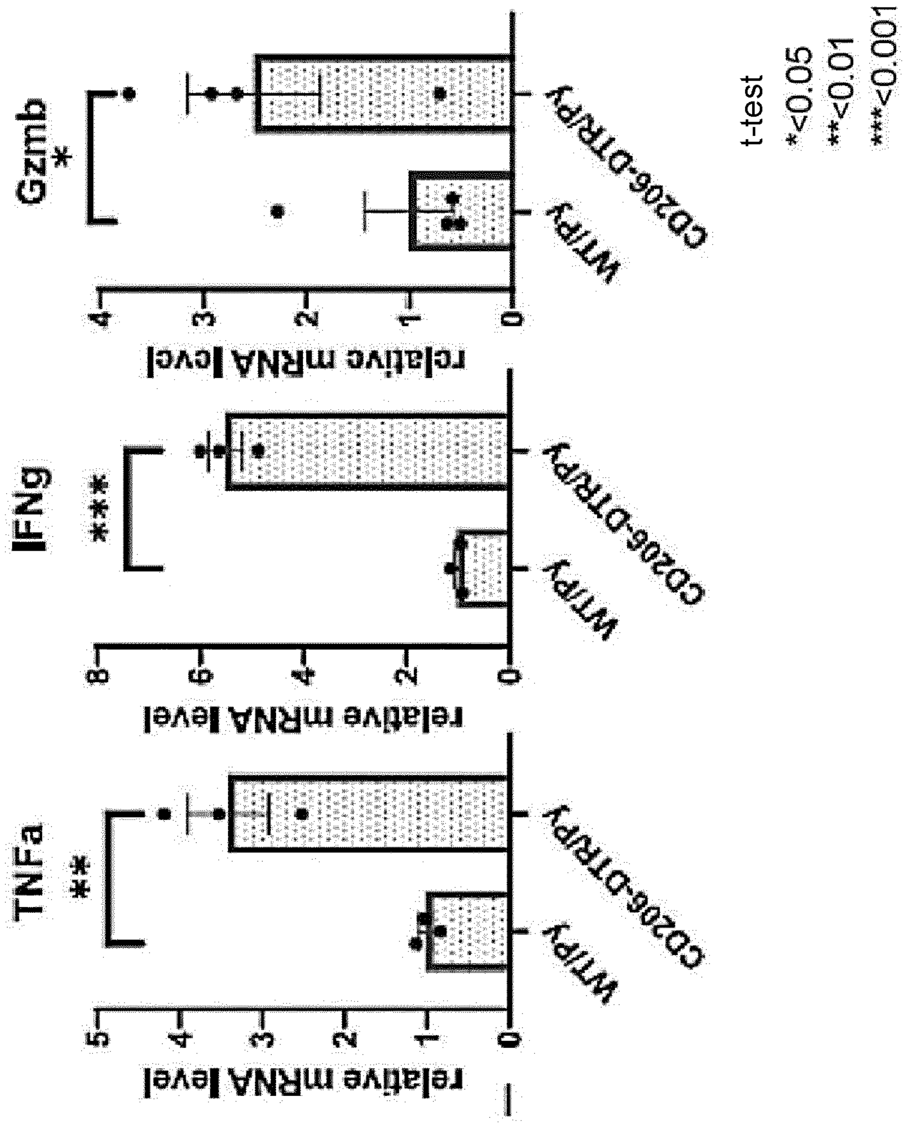

Fig. 4

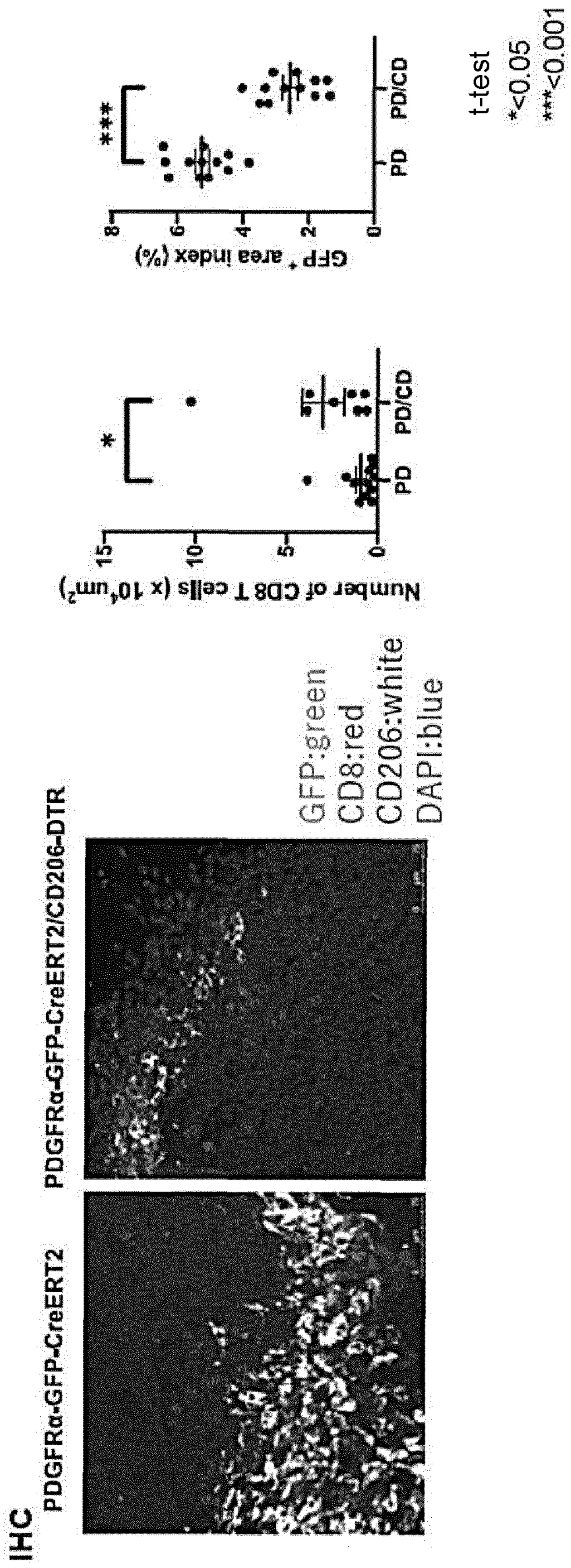
IHC
PDGFRα-GFP-CreERT2
PDGFRα-GFP-CreERT2/CD206-DTR
GFP:green
CD8:red
CD206:white
DAPI:blue
Number of CD8 T cells (x 10⁴um²)
15
10
5
0
PD
PD/CD
*
GFP + area index (%)
8
6
4
2
0
PD
PD/CD
***
t-test
*<0.05
***<0.001

Fig. 5

Quantitative RT-PCR analysis

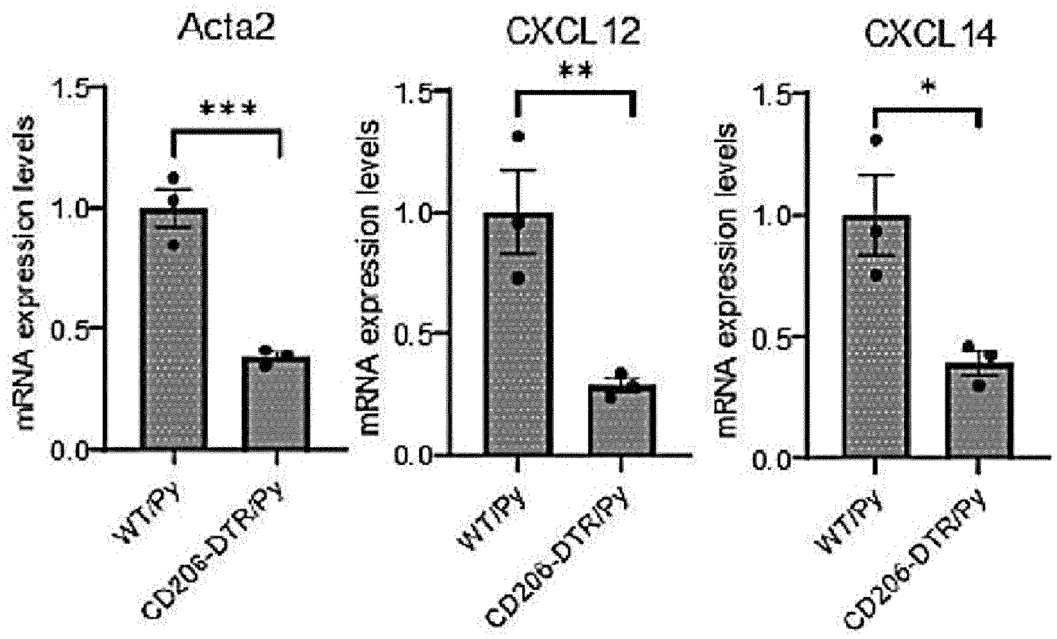


t-test
*<0.05
**<0.01
***<0.001

Fig. 6

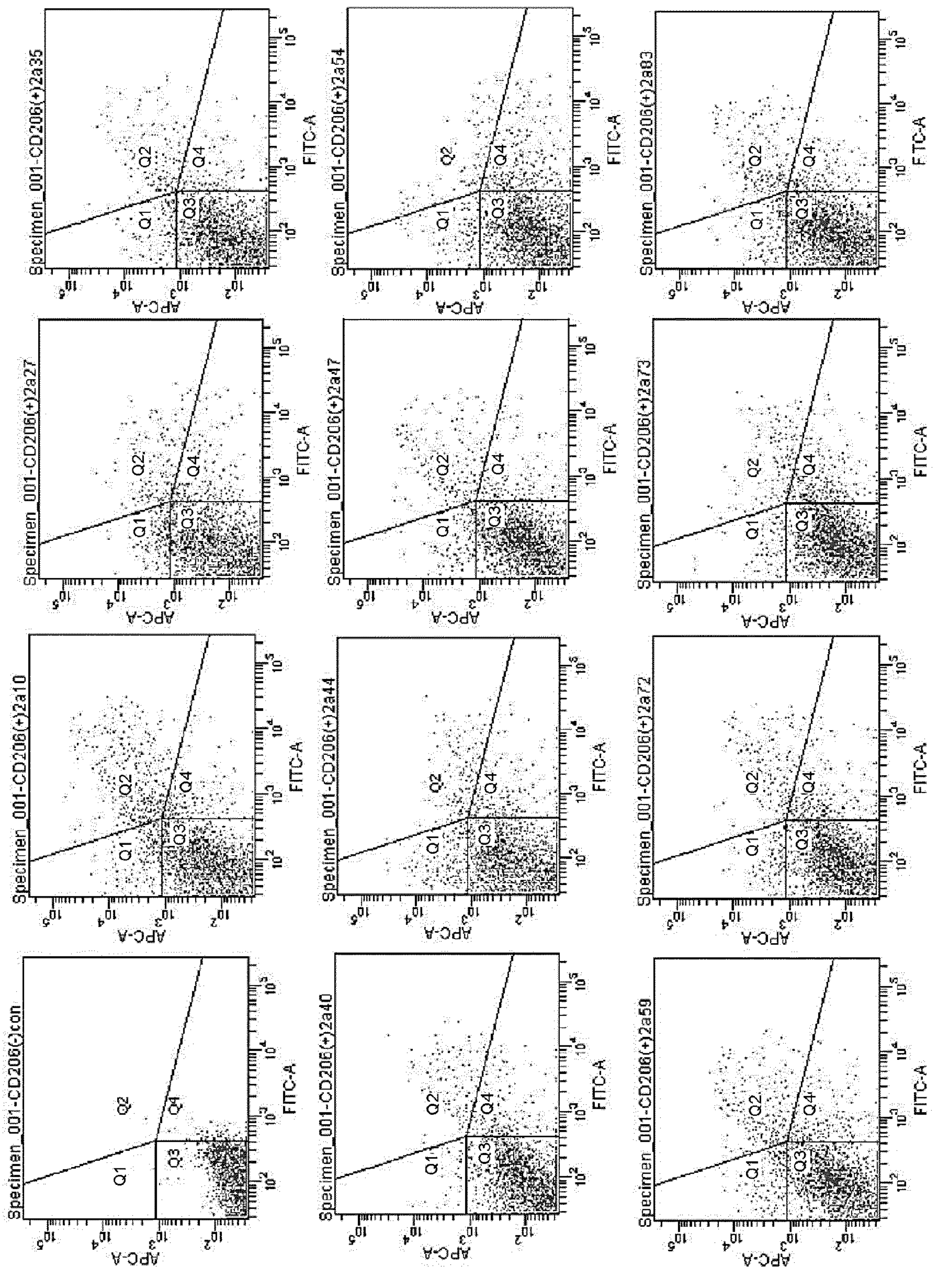
Specimen_001-CD206(-)con
Specimen_001-CD206(+)2a10
Specimen_001-CD206(+)2a27
Specimen_001-CD206(+)2a35
Specimen_001-CD206(+)2a40
Specimen_001-CD206(+)2a44
Specimen_001-CD206(+)2a47
Specimen_001-CD206(+)2a54
Specimen_001-CD206(+)2a59
Specimen_001-CD206(+)2a72
Specimen_001-CD206(+)2a73
Specimen_001-CD206(+)2a83
Q1
Q2
Q3
Q4
APC-A
FITC-A

Fig. 7

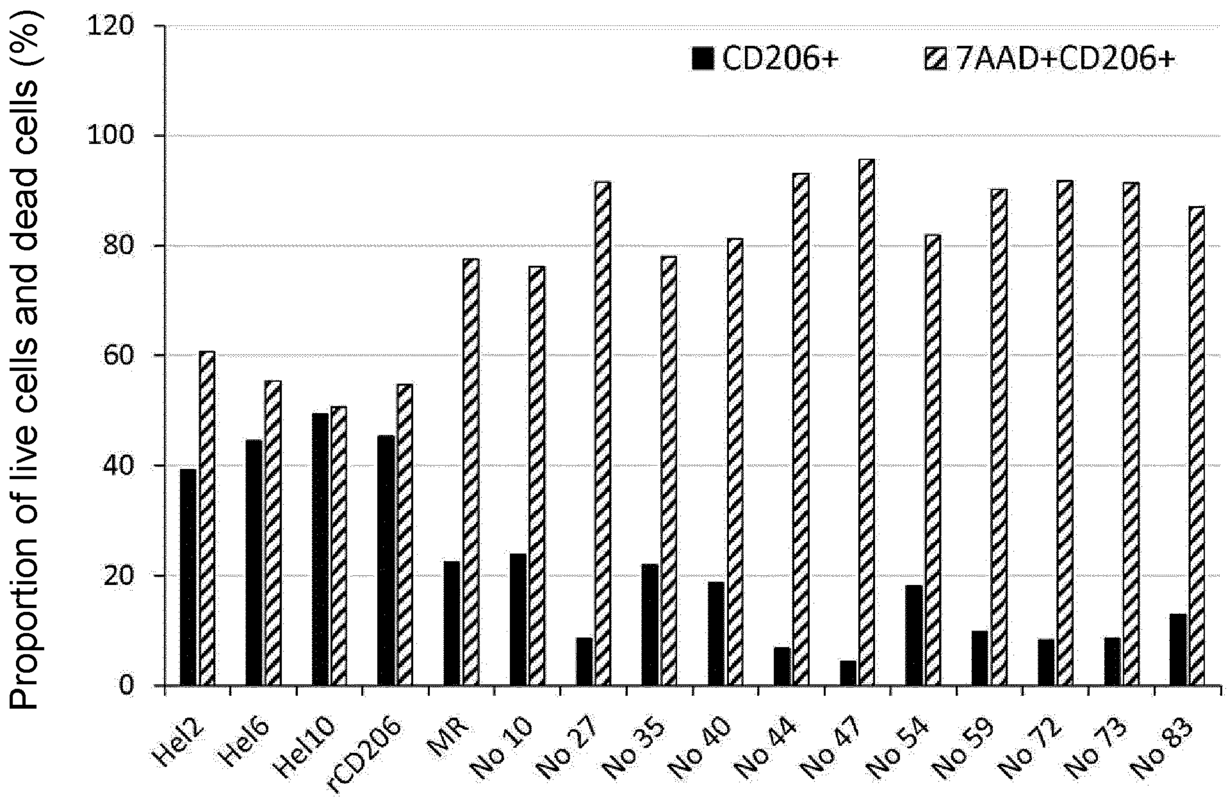
Proportion of live cells and dead cells (%)
120
100
80
60
40
20
0
CD206+
7AAD+CD206+
Hel2
Hel6
Hel10
rCD206
MR
No 10
No 27
No 35
No 40
No 44
No 47
No 54
No 59
No 72
No 73
No 83

Fig. 8

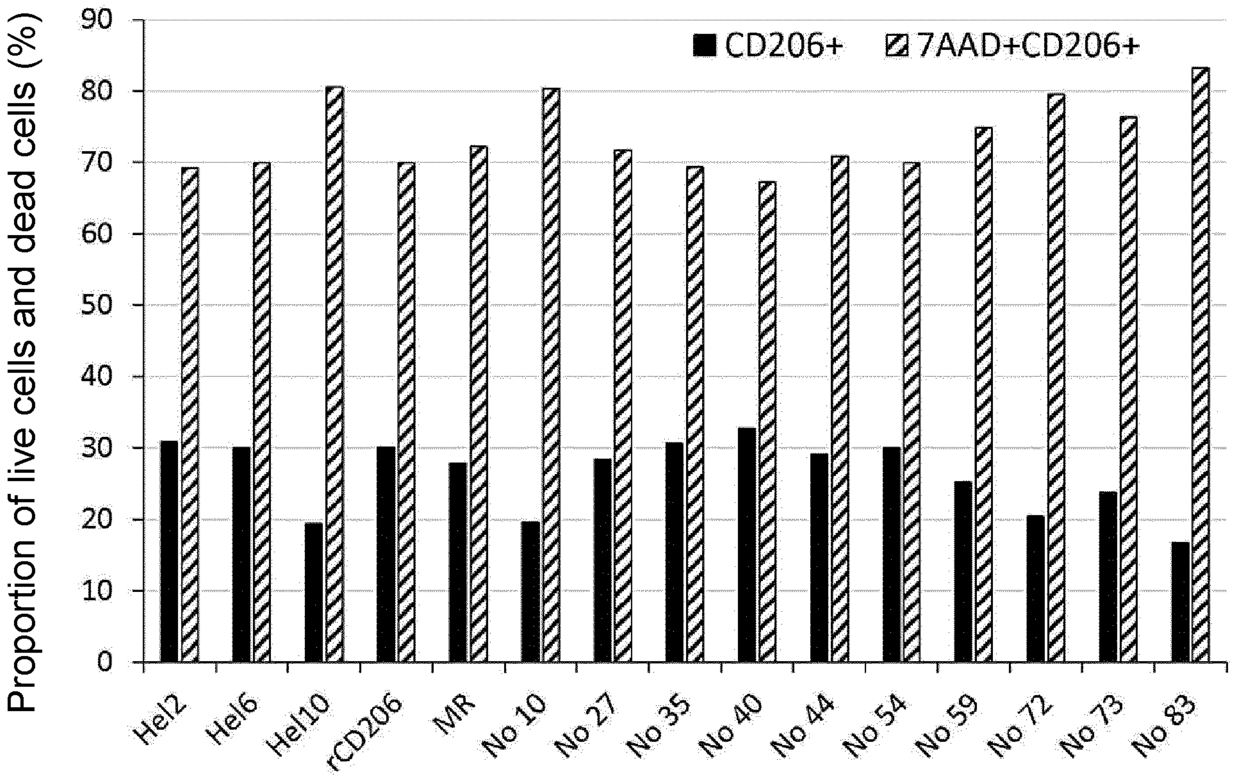
Proportion of live cells and dead cells (%)
0
10
20
30
40
50
60
70
80
90
CD206+
7AAD+CD206+
Hel2
Hel6
Hel10
rCD206
MR
No 10
No 27
No 35
No 40
No 44
No 54
No 59
No 72
No 73
No 83

Fig. 9

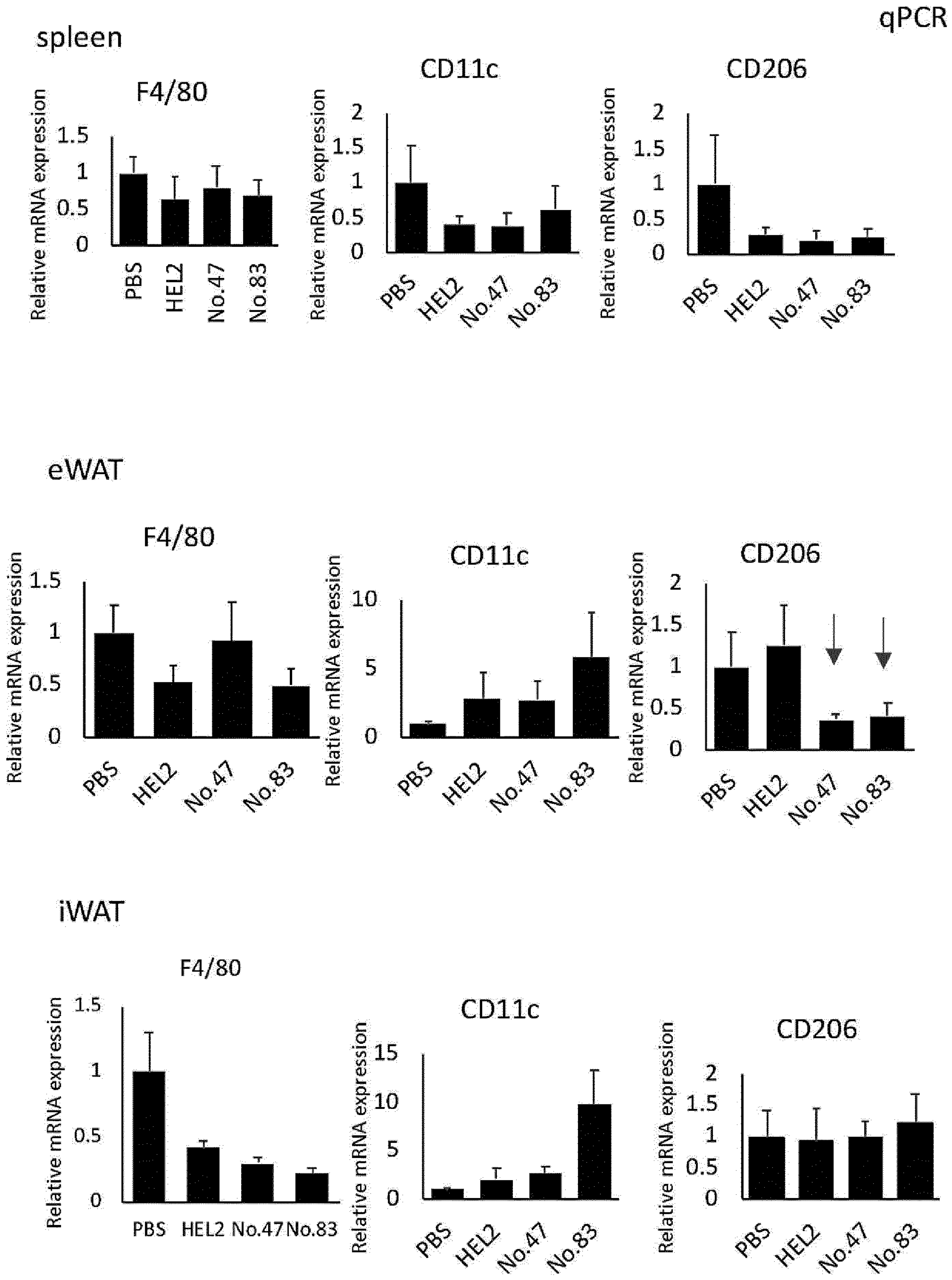
qPCR
spleen
F4/80
CD11c
CD206
eWAT
F4/80
CD11c
CD206
iWAT
F4/80
CD11c
CD206
Relative mRNA expression
PBS
HEL2
No.47
No.83

Fig. 10

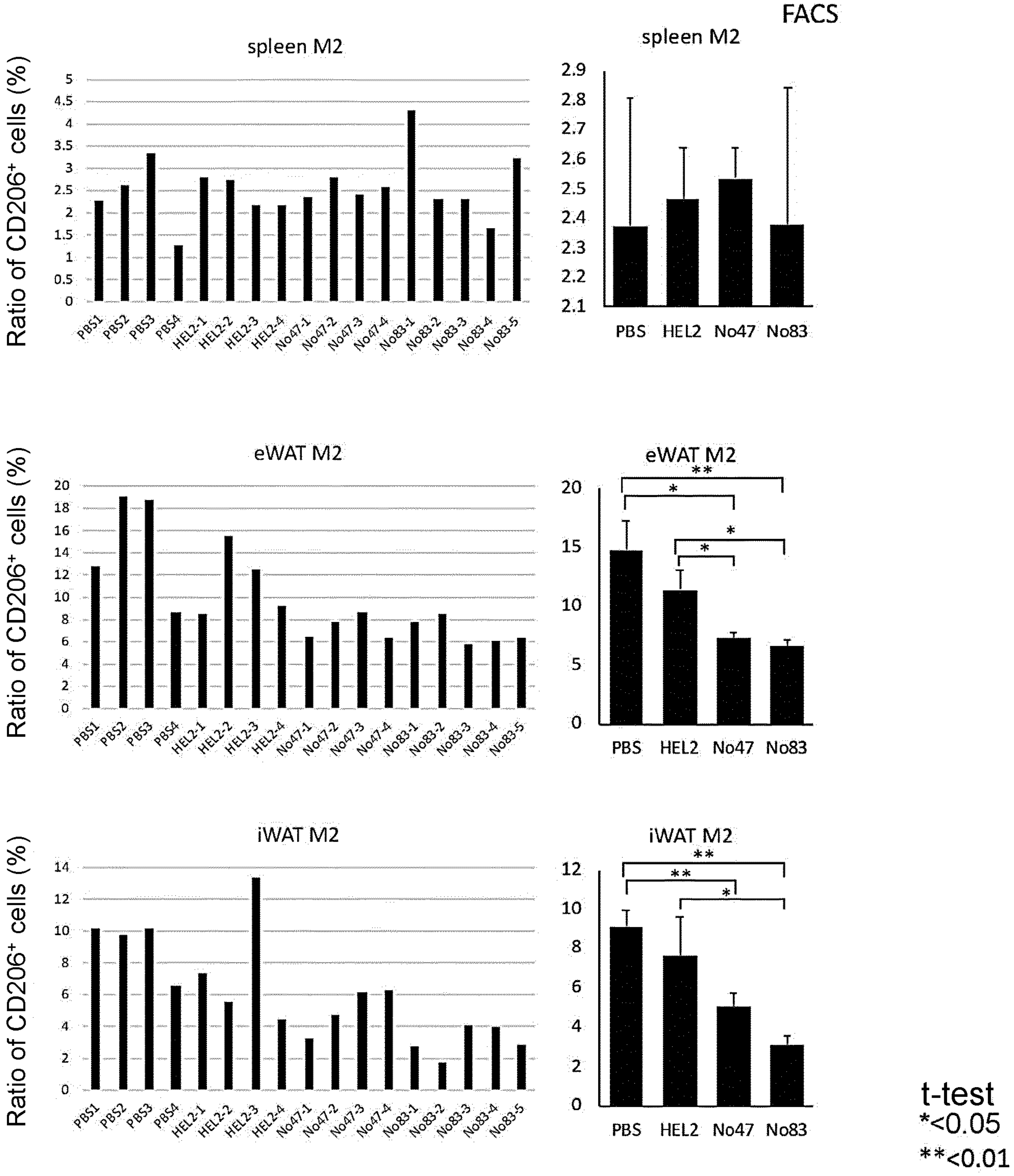
FACS
spleen M2
Ratio of CD206+ cells (%)
PBS1 PBS2 PBS3 PBS4 HEL2-1 HEL2-2 HEL2-3 HEL2-4 No47-1 No47-2 No47-3 No47-4 No83-1 No83-2 No83-3 No83-4 No83-5
PBS HEL2 No47 No83
eWAT M2
iWAT M2
t-test
*<0.05
**<0.01

## INTERNATIONAL SEARCH REPORT

International application No.
**PCT/JP2022/020881**

**A. CLASSIFICATION OF SUBJECT MATTER**

***A61K 45/00***(2006.01)i; ***A61K 39/395***(2006.01)i; ***A61P 35/00***(2006.01)i; ***A61P 43/00***(2006.01)i; ***C07K 16/28***(2006.01)i; *C07K 16/46*(2006.01)n; *C12N 15/13*(2006.01)n; *C12P 21/08*(2006.01)n

FI: A61K45/00 ZNA; A61K39/395 N; A61P35/00; A61P43/00 105; A61P43/00 111; C07K16/28; C07K16/46; C12N15/13; C12P21/08

According to International Patent Classification (IPC) or to both national classification and IPC

**B. FIELDS SEARCHED**

Minimum documentation searched (classification system followed by classification symbols)

A61K45/00; A61K39/395; A61P35/00; A61P43/00; C07K16/28; C07K16/46; C12N15/13; C12P21/08

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/MEDLINE/EMBASE/BIOSIS (STN), JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X | WO 2020/210625 A1 (MERIDIAN BIOSCIENCE, INC.) 15 October 2020 (2020-10-15) claims 1, 5-7, 15, paragraphs [0007], [0174], [0271]-[0284], [0317], [0329]-[0333], [0338], [0350], fig. 21A, 83 | 2-3, 5-11 |
| A | | 1, 4 |
| X | WO 2019/059267 A1 (DAIICHI SANKYO CO., LTD.) 28 March 2019 (2019-03-28) claims 3-6, paragraphs [0002], [0004], [0005], [0007], [0024], examples 3, 7 | 8–11 |
| A | | 1-7 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **30 June 2022** | **19 July 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2022/020881**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/JP2022/020881**

| Patent document cited in search report | Publication date (day/month/year) | Patent family member(s) | Publication date (day/month/year) |
|---|---|---|---|
| WO 2020/210625 A1 | 15 October 2020 | KR 10-2021-0151945 A<br>JP 2022-527133 A | |
| WO 2019/059267 A1 | 28 March 2019 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2009034047 A
- JP 2014073100 A
- JP 2014162772 A


### Non-patent literature cited in the description


- *Nat Rev Immunol.,* 2019, vol. 19 (6), 369-382
- **AISHUN JIN et al.** *Nature Medicine,* 09 September 2009, vol. 15, 1088-1093
- **AISHUN JIN et al.** *Nature Protocols,* 2011, vol. 6 (5), 668-676
- *Nat. Commun.,* 2017, vol. 8 (554), 1-15